(19) Europäisches Patentamt
European Patent Office
Office européen des brevets

(11) Veröffentlichungsnummer: **0 459 465 A2**

# (12) EUROPÄISCHE PATENTANMELDUNG

(21) Anmeldenummer: **91108828.4**

(51) Int. Cl.[5]: **C07K 5/02**, A61K 37/64

(22) Anmeldetag: **29.05.91**

(30) Priorität: **01.06.90 CH 1860/90**

(43) Veröffentlichungstag der Anmeldung:
**04.12.91 Patentblatt 91/49**

(84) Benannte Vertragsstaaten:
**AT BE CH DE DK ES FR GB GR IT LI LU NL SE**

(71) Anmelder: **CIBA-GEIGY AG**
**Klybeckstrasse 141**
**CH-4002 Basel(CH)**

(72) Erfinder: **Schneider, Peter, Dr.**
**Bäumliackerstrasse 8**
**CH-4103 Bottmingen(CH)**
Erfinder: **Fässler, Alexander, Dr.**
**Hallenstrasse 10**
**CH-4104 Oberwil(CH)**
Erfinder: **Lang, Marc, Dr.**
**Rue des Ormes 6**
**F-68170 Rixheim(FR)**

(74) Vertreter: **Zumstein, Fritz, Dr. et al**
**Zumstein & Klingseisen Patentanwälte**
**Bräuhausstrasse 4**
**W-8000 München 2(DE)**

## (54) HIV-Protease-Inhibitoren und Verfahren zu ihrer Herstellung.

(57) Die Erfindung betrifft Verbindungen der Formel

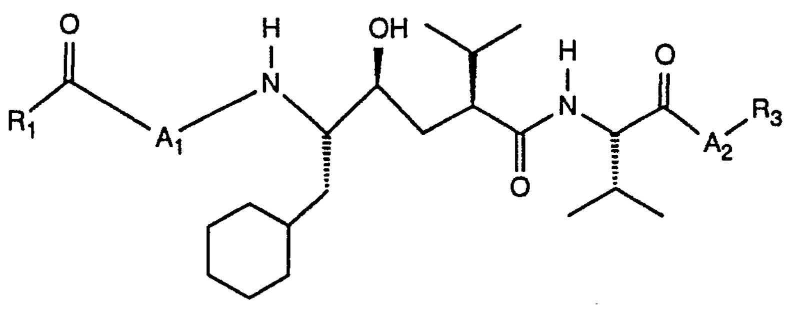

(I),

worin $R_1$ Diniederalkylamino, Pyrrolidino, Piperidino, Morpholino, Thiomorpholino, S,S-Dioxothiomorpholino, Piperazino, N-Niederalkylpiperazino, Benzyloxy oder im Phenylring durch Niederalkyl oder Niederalkoxy substituiertes Benzyloxy, $A_1$ ein bivalentes Radikal aus einer der Aminosäuren Valin oder Asparagin, das N-terminal an die Gruppe -(C=O) und C-terminal an die Gruppe -NH gebunden ist, $A_2$ ein bivalentes Radikal aus einer der Aminosäuren Tyrosin oder Phenylalanin, das N-terminal an die Gruppe -(C=O) und C-terminal an die Gruppe -NH gebunden ist, und $R_3$ $OR_2$ oder $NHR_2$, worin $R_2$ Wasserstoff oder Niederalkyl ist, bedeuten, ferner Salze dieser Verbindungen, sofern eine oder mehrere salzbildende Gruppen vorliegen. Die Verbindungen sind gag-Protease-Inhibitoren und geeignet zur Behandlung retroviraler Erkrankungen.

EP 0 459 465 A2

HIV (human immunodeficiency virus) ist ein Retrovirus, das die beim Menschen im allgemeinen tödlich verlaufende Krankheit AIDS verursacht. Während es heute möglich ist, die Auswirkungen von AIDS zu lindern und das Leben der Patienten zu verlängern, fehlen bisher pharmazeutische Präparate, die die dieses Virus als Ursache von AIDS wirksam bekämpfen. Eine mögliche Zielrichtung bei der Therapie von AIDS besteht darin, eine Vermehrung von HIV zu verhindern, ohne gleichzeitig die noch intakten Zellverbände des Patienten zu schädigen.

Das Genom der bislang bekannten beiden Varianten von HIV, HIV-1 und HIV-2, weist jeweils einen Bereich auf, der eine "gag-Protease" kodiert. Des weiteren enthalten beide Viren einen Bereich, der für die Gruppen-spezifischen Antigene, englisch "group specific antigen" (gag), kodiert. Die entsprechenden Gene werden als Vorläuferprotein exprimiert und daraus proteolytisch von der oben erwähnten gag-Protease die eigentlichen gag-Proteine, die Strukturproteine des Viruskerns p17, p24, p9 und p7, freigesetzt. Auch die gag-Protease selbst wird aus einem Vorläuferprotein herausgeschnitten, aus dem noch weitere virale Proteine, wie die Reverse Transcriptase und die Integrase, entstehen. Dieser Prozess verläuft vermutlich autoproteolytisch. Es ist ferner bekannt, dass die gag-Protease das "major core protein" (p24) von HIV-1 bzw. HIV-2 vorzugsweise N-terminal bei Prolin spaltet, z.B. bei Phe-Pro, Leu-Pro oder Tyr-Pro. Ein auf diese Weise verkürztes, Pro-terminales p24 dient dann zusammen mit weiteren viralen Strukturproteinen zum Aufbau des Nucleocapsids und der Virushülle von HIV-1 und HIV-2. Des weiteren ist bekannt, dass die HIV-1 gag-Protease Leu-Phe-, Leu-Ala-, Met-Met- und Phe-Leu-Sequenzen mit unterschiedlicher Effizienz spaltet. Es gilt als sicher, dass zusätzlich zur Aminosäuresequenz die Konformation zur Spezifität der gag-Protease beiträgt.

Wenn die Wirkung der gag-Protease unterbunden werden könnte, stünden dem Virus weder funktionelle Strukturproteine noch die notwendigen viralen Enzyme zur Verfügung, und seine Vermehrung wäre behindert, wenn nicht gar unterbrochen. Es besteht daher ein Bedürfnis nach Inhibitoren oder zumindest Hemmern der gag-Protease. Derartigen Verbindungen kommt eine wichtige Rolle als antivirale Mittel gegen AIDS oder andere retrovirale Erkrankungen zu.

Es wurde nun überraschenderweise gefunden, dass die erfindungsgemässen Verbindungen als gag-Protease-Inhibitoren geeignet sind, da sie imstande sind, gag-Proteasen bereits im nanomolaren Konzentrationsbereich zu hemmen.

Bei den erfindungsgemässen Verbindungen handelt es sich um die Verbindungen der Formel

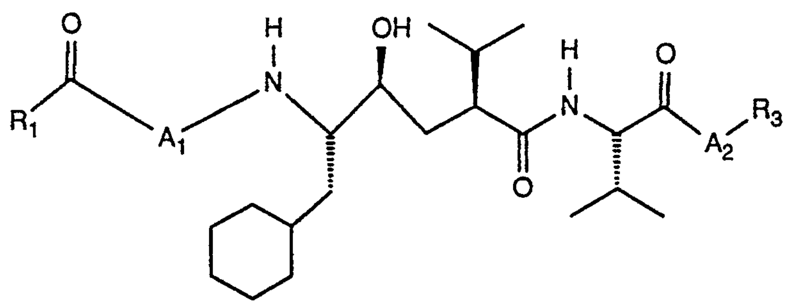

(I),

worin $R_1$ Diniederalkylamino, Pyrrolidino, Piperidino, Morpholino, Thiomorpholino, S,S-Dioxothiomorpholino, Piperazino, N-Niederalkylpiperazino, Benzyloxy oder im Phenylring durch Niederalkyl oder Niederalkoxy substituiertes Benzyloxy, $A_1$ ein bivalentes Radikal aus einer der Aminosäuren Valin oder Asparagin, das N-terminal an die Gruppe -(C=O) und C-terminal an die Gruppe -NH gebunden ist, $A_2$ ein bivalentes Radikal aus einer der Aminosäuren Tyrosin oder Phenylalanin, das N-terminal an die Gruppe -(C=O) und C-terminal an die Gruppe -NH gebunden ist, und $R_3$ $OR_2$ oder $NHR_2$, worin $R_2$ Wasserstoff oder Niederalkyl ist, bedeuten, ferner Salze dieser Verbindungen, sofern eine oder mehrere salzbildende Gruppen vorliegen..

Die Erfindung betrifft ferner Verfahren zur Herstellung der genannten Verbindungen, pharmazeutische Präparate enthaltend diese Verbindungen und die Verwendung dieser Verbindungen als Arzneimittel oder zur Herstellung von pharmazeutischen Präparaten.

In der Beschreibung der vorliegenden Erfindung bedeutet der Ausdruck "Nieder", dass die so definierten Gruppen oder Reste, z.B. Niederalkyl oder Niederalkoxy, vorzugsweise 1 bis 7 C-Atome und insbesondere 1 bis 4 C-Atome enthalten. Die allgemeinen Ausdrücke und Bezeichnungen haben die folgenden Bedeutungen:

Niederalkyl hat vorzugsweise 1 bis 7 C-Atome und ist beispielsweise Methyl, Ethyl, n-Propyl, Isopropyl, n-Butyl, Isobutyl, n-Pentyl oder n-Hexyl. Niederalkyl $R_2$ ist in $OR_2$ insbesondere Methyl, Ethyl oder n-Butyl, in

$NHR_2$ insbesondere n-Propyl. Niederalkyl in Diniederalkylamino $R_1$ ist insbesondere Methyl oder Ethyl, vor allem Methyl. Bevorzugter Substituent Niederalkyl in Benzyloxy $R_1$ ist Methyl.

Niederalkoxy hat vorzugsweise 1 bis 7 C-Atome und ist beispielsweise Methoxy, Ethoxy, Isopropoxy oder tert-Butoxy, insbesondere Methoxy.

S,S-Dioxothiomorpholino bedeutet den über Stickstoff gebundenen Rest des von Thiomorpholin abgeleiteten Sulfons, 4-Aza-1,1-Dioxo-1-thiacyclohex-4-yl.

Der Substituent Niederalkyl oder Niederalkoxy in Benzyloxy $R_1$ steht in 2-, 3- oder 4-Stellung des Phenylrings, vorzugsweise in 4-Stellung, und kann auch mehrfach vorkommen. Substituiertes Benzyloxy $R_1$ ist beispielsweise 2-, 3- oder 4-Methylbenzyloxy, 2-, 3- oder 4-Methoxybenzyloxy, 2,4- oder 3,5-Dimethylbenzyloxy oder 2,4-Dimethoxybenzyloxy.

Valin ($\alpha$-Amino-isovaleriansäure) und Asparagin ($\alpha$-Amino-bernsteinsäure-4-amid) $A_1$ oder Phenylalanin ($\alpha$-Amino-$\beta$-phenylpropionsäure) und Tyrosin ($\alpha$-Amino-$\beta$-(p-hydroxyphenyl)propionsäure) $A_2$ bezeichnen die entsprechenden in natürlichen Proteinen vorkommenden L-$\alpha$-Aminosäuren.

Salze sind in erster Linie die pharmazeutisch verwendbaren, nichttoxischen Salze von Verbindungen der Formel I, die eine oder mehrere salzbildende saure oder basische Gruppen enthalten.

Solche Salze werden beispielsweise von Verbindungen der Formel I, worin $R_2$ Wasserstoff bedeutet, gebildet und sind in erster Linie geeignete Alkalimetall-, z.B. Natrium- oder Kalium-, oder Erdalkalimetallsalze, z.B. Magnesium- oder Calciumsalze, ferner Zinksalze oder Ammoniumsalze, auch solche Salze, welche mit organischen Aminen, wie gegebenenfalls durch Hydroxy substituierten Mono-, Di- oder Trialkylaminen, gebildet werden, z.B. mit Diethylamin, Di-(2-hydroxyethyl)-amin, Triethylamin, N,N-Dimethyl-N-(2-hydroxyethyl)-amin, Tri-(2-hydroxyethyl)-amin oder N-Methyl-D-glucamin. Die Verbindungen der Formel I mit einer basischen Gruppe, z.B. einer Aminogruppe, können Säureadditionssalze bilden, z.B. mit anorganischen Säuren, z.B. Salzsäure, Schwefelsäure oder Phosphorsäure, oder mit organischen Carbon-, Sulfon- oder Sulfosäuren, z.B. Essigsäure, Propionsäure, Glykolsäure, Bernsteinsäure, Maleinsäure, Hydroxymaleinsäure, Methylmaleinsäure, Fumarsäure, Aepfelsäure, Weinsäure, Zitronensäure, Benzoesäure, Zimtsäure, Mandelsäure, Salicylsäure, 4-Aminosalicylsäure, 2-Phenoxybenzoesäure, 2-Acetoxybenzoesäure, Embonsäure, Nicotinsäure oder Isonicotinsäure, ferner mit $\alpha$-Aminosäuren, sowie mit Methansulfonsäure, Ethansulfonsäure, 2-Hydroxyethansulfonsäure, Ethan-1,2-disulfonsäure, Benzolsulfonsäure, 4-Methylbenzolsulfonsäure oder Naphthalin-2-sulfonsäure, oder mit anderen sauren organischen Verbindungen, wie Ascorbinsäure. Verbindungen der Formel I mit sauren und basischen Gruppen können auch innere Salze bilden.

Zur Isolierung oder Reinigung können auch pharmazeutisch ungeeignete Salze Verwendung finden.

Die Verbindungen der vorliegenden Erfindung weisen gag-Protease-hemmende Wirkungen auf. Insbesondere hemmen sie in Konzentrationen bis hinunter zum nanomolaren Bereich die Wirkung der gag-Protease von HIV-1 und HIV-2 und sind daher geeignet als Mittel gegen durch diese oder verwandte Retroviren verursachte Krankheiten, wie z.B. gegen AIDS.

Die Fähigkeit der Verbindungen der Formel I, die proteolytische Aktivität von z.B. HIV-1 Protease zu inhibieren, lässt sich beispielsweise gemäss dem von J. Hansen et al., The EMBO Journal 7,1785-1791-(1988), beschriebenen Verfahren demonstrieren. Dabei wird die Hemmung der Wirkung der gag-Protease auf ein Substrat gemessen, das ein in E. coli exprimiertes Fusionsprotein aus dem gag-Vorläuferprotein und MS-2 ist. Das Substrat und seine Spaltprodukte werden durch Polyacrylamid-Gelektrophorese getrennt und durch Immunoblotting mit monoklonalen Antikörpern gegen MS-2 sichtbar gemacht.

In einem noch einfacher zu handhabenden Test, der genaue quantitative Aussagen ermöglicht, wird als Substrat für die gag-Protease ein synthetisches Icosapeptid eingesetzt, das der Spaltstelle des gag-Vorläuferproteins entspricht. Dieses Substrat und seine Spaltprodukte können durch Hochdruckflüssig-Chromatographie (HPLC) gemessen werden. Verbindungen der vorliegenden Erfindung zeigen in diesem Test Hemmwirkungen in Konzentrationen von $10^{-6}$ mol/l.

In einem weiteren Test kann gezeigt werden, dass die Verbindungen der vorliegenden Erfindung Zellen, die normalerweise von HIV infiziert werden, vor einer solchen Infektion schützen oder zumindest eine solche Infektion verlangsamen. Dabei wird die menschliche T-Zell-Leukämie Zellinie MT-2 (Science 229, 563 (1985)), die extrem empfindlich für den zytopathogenen Effekt von HIV ist, mit HIV allein oder mit HIV in Gegenwart der erfindungsgemässen Verbindungen inkubiert und nach einigen Tagen die Lebensfähigkeit der so behandelten Zellen beurteilt. Erfindungsgemässe Verbindungen zeigen Infektions-hemmende Wirkungen in Konzentrationen von $10^{-5}$ mol/l.

Bevorzugt sind Verbindungen der Formel I, worin $R_1$ Diniederalkylamino, Piperidino, Morpholino, Thiomorpholino, S,S-Dioxothiomorpholino oder Benzyloxy, $A_1$ den bivalenten Rest der Aminosäure Valin, $A_2$ den bivalenten Rest der Aminosäure Tyrosin, und $R_3$ $OR_2$, worin $R_2$ Niederalkyl ist, bedeuten.

Bevorzugt sind auch Verbindungen der Formel I, worin $R_1$ Diniederalkylamino, Piperidino, Morpholino, Thiomorpholino, S,S-Dioxothiomorpholino oder Benzyloxy, $A_1$ den bivalenten Rest der Aminosäure Aspara-

gin oder Valin, $A_2$ den bivalenten Rest der Aminosäure Phenylalanin oder Tyrosin, und $R_3$ $OR_2$, worin $R_2$ Niederalkyl ist, oder $NHR_2$, worin $R_2$ Wasserstoff oder Niederalkyl ist, bedeuten.

Ganz besonders bevorzugt sind Verbindungen der Formel I, worin $R_1$ Morpholino, Thiomorpholino, S,S-Dioxothiomorpholino oder Benzyloxy, $A_1$ den bivalenten Rest der Aminosäure Valin, $A_2$ den bivalenten Rest der Aminosäure Tyrosin, und $R_3$ $OR_2$, worin $R_2$ Niederalkyl, insbesondere Methyl, ist, bedeuten.

Ganz besonders bevorzugt sind auch die Verbindungen der Formel I, worin $R_1$ Morpholino oder Benzyloxy, $A_1$ den bivalenten Rest der Aminosäure Asparagin, $A_2$ den bivalenten Rest der Aminosäure Tyrosin, und $R_3$ $OR_2$, worin $R_2$ Niederalkyl, insbesondere Methyl, ist, bedeuten.

Ebenfalls ganz besonders bevorzugt sind Verbindungen der Formel I, worin $R_1$ Morpholino, $A_1$ den bivalenten Rest der Aminosäure Valin oder Asparagin, $A_2$ den bivalenten Rest der Aminosäure Phenylalanin oder Tyrosin, und $R_3$ $NHR_2$, worin $R_2$ Wasserstoff oder Niederalkyl, insbesondere Wasserstoff oder n-Propyl, ist, bedeuten.

Die erfindungsgemässen Verbindungen der Formel I und Salze von solchen Verbindungen mit mindestens einer salzbildenden Gruppe werden nach an sich bekannten Verfahren erhalten, z.B. indem man

ein Fragment einer Verbindung der Formel I mit einer endständigen Carboxygruppe oder ein reaktionsfähiges Säurederivat dieses Fragments mit einem zur Verbindung der Formel I komplementären Fragment mit einer freien Aminogruppe oder einem reaktionsfähigen Derivat davon mit aktivierter Aminogruppe, wobei in den Reaktionskomponenten vorhandene funktionelle Gruppen mit Ausnahme der an der Reaktion teilnehmenden Gruppen gegebenenfalls in geschützter Form vorliegen, unter Bildung einer Amidbindung kondensiert, und gewünschtenfalls

in einer erhältlichen Verbindung vorhandene Schutzgruppen abspaltet und/oder eine erhältliche Verbindung der Formel I mit einer salzbildenden Gruppe in ihr Salz oder ein erhältliches Salz in die freie Verbindung oder in ein anderes Salz überführt und/oder gegebenenfalls erhältliche Isomerengemische auftrennt und/oder eine erfindungsgemässe Verbindung der Formel I in eine andere erfindungsgemässe Verbindung der Formel I umwandelt.

Fragmente einer Verbindung der Formel I mit einer endständigen Carboxygruppe, welche mit einem zur Verbindung der Formel I komplementären Fragment unter Bildung einer Amidbindung kondensiert werden können, sind z.B. Verbindungen der Formeln

$R_1$-COOH (II),

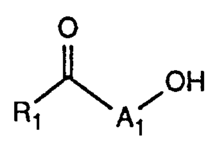

(III),

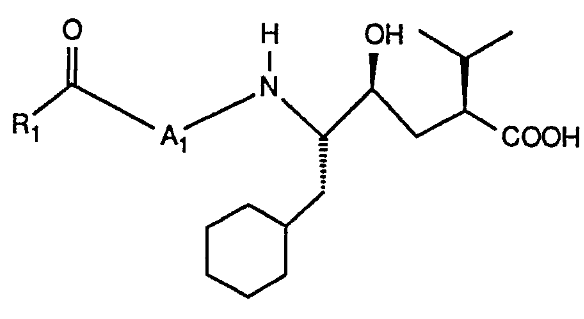

(IV),

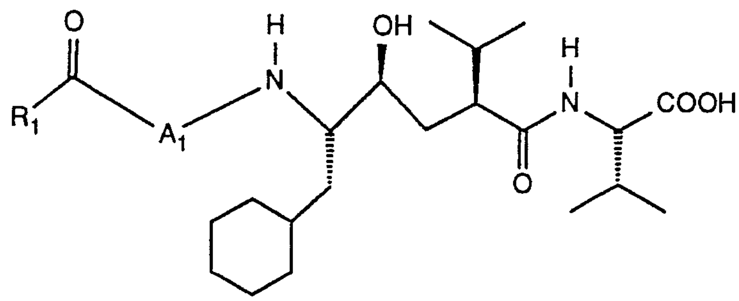

(V), und

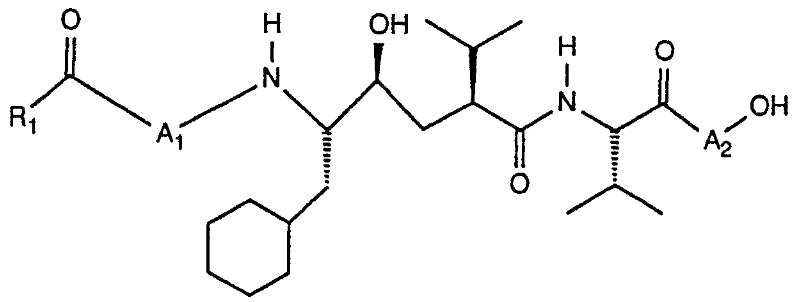

(VI),

worin die Reste die genannten Bedeutungen haben, die von diesen Verbindungen abgeleiteten aktivierten Ester oder reaktionsfähigen Anhydride, ferner reaktionsfähige cyclische Amide. Die reaktionsfähigen Säurederivate können auch in situ gebildet werden.

Aktivierte Ester sind insbesondere am Verknüpfungskohlenstoffatom des veresternden Restes ungesättigte Ester, z.B. vom Vinylester-Typ, wie Vinylester (erhältlich z.B. durch Umesterung eines entsprechenden Esters mit Vinylacetat; Methode des aktivierten Vinylesters), Carbamoylvinylester (erhältlich z.B. durch Behandeln der entsprechenden Säure mit einem Isoxazoliumreagens; 1,2-Oxazolium- oder Woodward-Methode) oder 1-Niederalkoxyvinylester (erhältlich z.B. durch Behandeln der entsprechenden Säure mit einem Niederalkoxyacetylen; Ethoxyacetylen-Methode), oder Ester vom Amidinotyp, wie N,N'-disubstituierte Amidinoester (erhältlich z.B. durch Behandeln der entsprechenden Säure mit einem geeigneten N,N'-disubstituierten Carbodiimid, z.B. N,N'-Dicyclohexylcarbodiimid; Carbodiimid-Methode) oder N,N-disubstituierte Amidinoester (erhältlich z.B. durch Behandeln der entsprechenden Säure mit einem N,N-disubstituierten Cyanamid; Cyanamid-Methode), geeignete Arylester, insbesondere durch elektronenanziehende Substituenten substituierte Phenylester (erhältlich z.B. durch Behandeln der entsprechenden Säure mit einem geeignet substituierten Phenol, z.B. 4-Nitrophenol, 4-Methylsulfonylphenol, 2,4,5-Trichlorphenol, 2,3,4,5,6-Pentachlorphenol oder 4-Phenyldiazophenol, in Gegenwart eines Kondensationsmittels, wie N,N'-Dicyclohexylcarbodiimid; Methode der aktivierten Arylester), Cyanmethylester (erhältlich z.B. durch Behandeln der entsprechenden Säure mit Chloracetonitril in Gegenwart einer Base; Cyanmethylester-Methode), Thioester, insbesondere gegebenenfalls, z.B. durch Nitro, substituierte Phenylthioester (erhältlich z.B. durch Behandeln der entsprechenden Säure mit gegebenenfalls, z.B. durch Nitro, substituierten Thiophenolen, u.a. mit Hilfe der Anhydrid- oder Carbodiimid-Methode; Methode der aktivierten Thiolester) oder insbesondere Amino- oder Amidoester (erhältlich z.B. durch Behandeln der entsprechenden Säure mit einer N-Hydroxyamino- bzw. N-Hydroxyamido-Verbindung und deren aktivierten Derivaten, z.B. N-Hydroxysuccinimid, N-Hydroxypiperidin, N-Hydroxyphthalimid, N-Hydroxy-5-norbornen- oder norbornan-2,3-dicarbonsäureimid, 1-Hydroxybenztriazol bzw. Benztriazol-1-yloxyphosphoniumsalzen oder O-Benztriazol-1-yluroniumsalzen, oder 3-Hydroxy-3,4-dihydro-1,2,3-benztriazin-4-on, z.B. nach der Anhydrid- oder Carbodiimid-Methode; Methode der aktivierten N-Hydroxyester).

Anhydride von Säuren können symmetrische oder vorzugsweise gemischte Anhydride dieser Säuren sein, z.B. Anhydride mit anorganischen Säuren, wie Säurehalogenide, insbesondere Säurechloride (erhältlich z.B. durch Behandeln der entsprechenden Säure mit Thionylchlorid, Phosphorpentachlorid oder Oxalylchlorid, oder gegebenenfalls Behandeln eines Alkohols oder Amins mit Phosgen; Säurechloridmethode), Azide (erhältlich z.B. aus einem entsprechenden Säureester über das entsprechende Hydrazid und dessen Behandlung mit salpetriger Säure; Azidmethode), Anhydride mit Kohlensäurehalbestern, z.B. Kohlensäureniederalkylhalbestern (erhältlich z.B. durch Behandeln der entsprechenden Säure mit Chlorameisensäureniederalkylestern oder mit einem 1-Niederalkoxycarbonyl-2-niederalkoxy-1,2-dihydrochinolin, z.B.

1-Ethoxycarbonyl-2-ethoxy-1,2-dihydrochinolin; Methode der gemischten O-Alkylkohlensäureanhydride), Anhydride mit dihalogenierter, insbesondere dichlorierter Phosphorsäure (erhältlich z.B. durch Behandeln der entsprechenden Säure mit Phosphoroxychlorid; Phosphoroxychloridmethode), Anhydride mit anderen Phosphorsäurederivaten (z.B. solchen, die man mit Phenyl-N-phenylphosphoramidochloridat erhalten kann) oder mit Phosphorigsäurederivaten, oder Anhydride mit organischen Säuren, wie gemischte Anhydride mit organischen Carbonsäuren (erhältlich z.B. durch Behandeln der entsprechenden Säure mit einem gegebenenfalls substituierten Niederalkan- oder Phenylniederalkancarbonsäurehalogenid, z.B. Phenylessigsäure-, Pivalinsäure- oder Trifluoressigsäurechlorid; Methode der gemischten Carbonsäureanhydride) oder mit organischen Sulfonsäuren (erhältlich z.B. durch Behandeln eines Salzes, wie eines Alkalimetallsalzes, der entsprechenden Säure mit einem geeigneten organischen Sulfonsäurehalogenid, wie Niederalkan- oder Aryl-, z.B. Methan- oder p-Toluolsulfonsäurechlorid; Methode der gemischten Sulfonsäureanhydride), sowie symmetrische Anhydride (erhältlich z.B. durch Kondensation der entsprechenden Säure in Gegenwart eines Carbodiimids oder von 1-Diethylaminopropin; Methode der symmetrischen Anhyride).

Geeignete cyclische Amide sind insbesondere Amide mit fünfgliedrigen Diazacyclen aromatischen Charakters, wie Amide mit Imidazolen, z.B. Imidazol (erhältlich z.B. durch Behandeln der entsprechenden Säure mit N,N'-Carbonyldiimidazol; Imidazol-Methode), oder Pyrazolen, z.B. 3,5-Dimethylpyrazol (erhältlich z.B. über das Säurehydrazid durch Behandeln mit Acetylaceton; Pyrazolid-Methode).

Zur Verbindung der Formel I komplementäre Fragmente mit einer freien Aminogruppe sind z.B. Verbindungen der Formeln

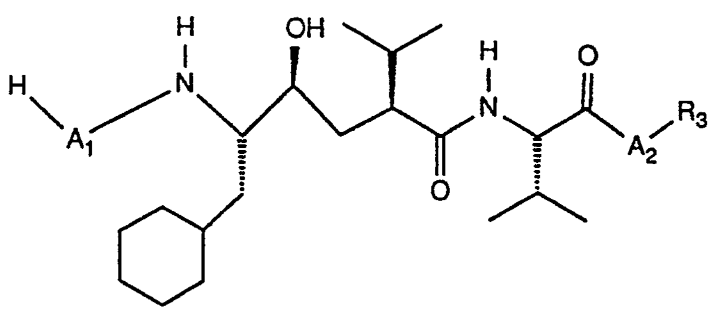

(VII),

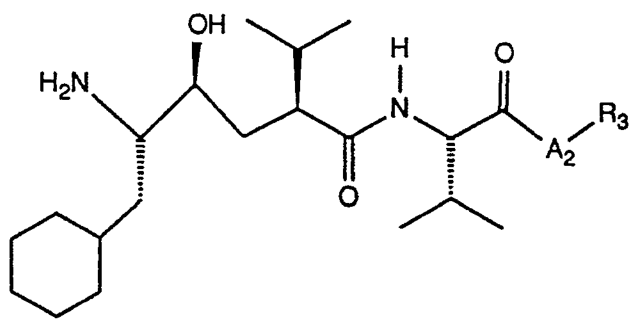

(VIII),

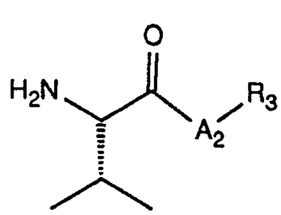

(IX),

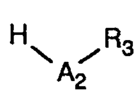

(X) und

$H_2N-R_2$ (XI) ,

worin die Reste die genannten Bedeutungen haben.

Die an der Reaktion teilnehmende Aminogruppe in einem zu einer Verbindung der Formel I komplementären Fragment liegt bevorzugt in freier Form vor, insbesondere wenn die damit reagierende Carboxygruppe in reaktionsfähiger Form vorliegt; sie kann aber auch selbst derivatisiert sein, z.B. durch Reaktion mit einem Phosphit, wie Diethylchlorphosphit, 1,2-Phenylenchlorphosphit, Ethyldichlorphosphit, Ethylenchlorphosphit oder Tetraethylpyrophosphit. Ein Derivat eines solchen komplementären Bruchstücks mit einer Aminogruppe ist z.B. auch ein Carbaminsäurehalogenid oder ein Isocyanat, wobei die an der Reaktion teilnehmende Aminogruppe durch Halogencarbonyl, z.B. Chlorcarbonyl, substituiert bzw. als Isocyanatgruppe abgewandelt ist.

Funktionelle Gruppen in Ausgangsmaterialien, deren Umsetzung vermieden werden soll, insbesondere Carboxy-, Amino- oder Hydroxygruppen, können durch geeignete Schutzgruppen geschützt sein, wie sie üblicherweise bei der Synthese von Peptid-Verbindungen, aber auch von Cephalosporinen und Penicillinen verwendet werden. Diese Schutzgruppen können bereits in den Vorstufen vorhanden sein und sollen die betreffenden funktionellen Gruppen gegen unerwünschte Nebenreaktionen wie Acylierungen, Veretherungen, Veresterungen, Oxidationen, Solvolyse etc. schützen. Schutzgruppen können aber auch in den Endstoffen vorhanden sein. Verbindungen der Formel I mit geschützten funktionellen Gruppen können eine höhere metabolische Stabilität aufweisen als die entsprechenden Verbindungen mit freien funktionellen Gruppen.

Der Schutz von funktionellen Gruppen durch solche Schutzgruppen, die Schutzgruppen selbst und ihre Abspaltungsreaktionen sind beispielsweise in Standardwerken wie in J.F.W. McOmie, "Protective Groups in Organic Chemistry", Plenum Press, London und New York 1973, in Th. W. Greene, "Protective Groups in Organic Synthesis", Wiley, New York 1981, in "The Peptides"; Band 3 (Herausg.. E. Gross und J. Meienhofer), Academic Press, London und New York 1981, sowie in "Methoden der organischen Chemie", Houben-Weyl, 4. Auflage, Bd. 15/I, Georg Thieme Verlag, Stuttgart 1974, beschrieben.

Eine Carboxygruppe ist z.B. als eine Estergruppe geschützt, die unter schonenden Bedingungen selektiv spaltbar ist. Eine in veresterter Form geschützte Carboxygruppe ist in erster Linie durch eine Niederalkylgruppe verestert, welche in 1-Stellung der Niederalkylgruppe verzweigt oder in 1- oder 2-Stellung der Niederalkylgruppe durch geeignete Substituenten substituiert ist.

Eine geschützte Carboxygruppe, welche durch eine Niederalkylgruppe verestert ist, die in 1-Stellung der Niederalkylgruppe verzweigt ist, ist beispielsweise tert-Niederalkoxycarbonyl, z.B. tert-Butoxycarbonyl, oder Arylmethoxycarbonyl mit einem oder zwei Arylresten, worin Aryl unsubstituiertes oder z.B. durch Niederalkyl, z.B. tert-Niederalkyl, wie tert-Butyl, Niederalkoxy, z.B. Methoxy, Hydroxy, Halogen, z.B. Chlor, und- oder Nitro mono-, di- oder trisubstituiertes Phenyl bedeutet, beispielsweise Benzyloxycarbonyl, durch die genannten Substituenten substituiertes Benzyloxycarbonyl, z.B. 4-Nitrobenzyloxycarbonyl oder 4-Methoxybenzyloxycarbonyl, Diphenylmethoxycarbonyl oder durch die genannten Substituenten substituiertes Diphenylmethoxycarbonyl, z.B. Di-(4-methoxyphenyl)-methoxycarbonyl.

Eine geschützte Carboxygruppe, welche durch eine Niederalkylgruppe verestert ist, welche in 1- oder 2-Stellung der Niederalkylgruppe durch geeignete Substituenten substituiert ist, ist beispielsweise 1-Niederalkoxyniederalkoxycarbonyl, z.B. Methoxymethoxycarbonyl, 1-Methoxyethoxycarbonyl oder 1-Ethoxyethoxycarbonyl, 1-Niederalkylthioniederalkoxycarbonyl, z.B. 1-Methylthiomethoxycarbonyl oder 1-Ethylthioethoxycarbonyl, Aroylmethoxycarbonyl, z.B. Phenacyloxycarbonyl, 2-Halogenniederalkoxycarbonyl, z.B. 2,2,2-Trichlorethoxycarbonyl, 2-Bromethoxycarbonyl oder 2-Iodethoxycarbonyl, sowie 2-Triniederalkylsilylniederalkoxycarbonyl, z.B. 2-Trimethylsilylethoxycarbonyl.

Eine Carboxygruppe kann auch als organische Silyloxycarbonylgruppe geschützt sein. Eine organische Silyloxycarbonylgruppe ist beispielsweise eine Triniederalkylsilyloxycarbonylgruppe, z.B. Trimethylsilyloxycarbonyl.

Eine geschützte Carboxygruppe ist bevorzugt tert-Niederalkoxycarbonyl, z.B. tert-Butoxycarbonyl, Benzyloxycarbonyl, 4-Nitrobenzyloxycarbonyl oder Diphenylmethoxycarbonyl.

Eine Aminogruppe kann z.B. in Form einer Acylamino-, Arylmethylamino-, veretherten Mercaptoamino- oder Silylaminogruppe geschützt sein.

In einer entsprechenden Acylaminogruppe ist Acyl beispielsweise der Acylrest einer organischen Carbonsäure mit z.B. bis zu 18 Kohlenstoffatomen, insbesondere einer gegebenenfalls, z.B. durch Halogen oder Aryl, substituierten Niederalkancarbonsäure, einer gegebenenfalls, z.B. durch Halogen, Niederalkoxy oder Nitro, substituierten Benzoesäure oder bevorzugt eines Kohlensäurehalbesters. Solche Acylgruppen sind beispielsweise Niederalkanoyl, wie Formyl, Acetyl, Propionyl oder Pivaloyl, Halogenniederalkanoyl, z.B. 2-Halogenacetyl, wie 2-Chlor-, 2-Brom-, 2-Iod-, 2,2,2-Trifluor- oder 2,2,2-Trichloracetyl, gegebenenfalls z.B.

durch Halogen, Niederalkoxy oder Nitro substituiertes Benzoyl, z.B. Benzoyl, 4-Chlorbenzoyl, 4-Methoxybenzoyl oder 4-Nitrobenzoyl, oder in 1-Stellung des Niederalkylrestes verzweigtes oder in 1- oder 2-Stellung geeignet substituiertes Niederalkoxycarbonyl, z.B. tert-Niederalkoxycarbonyl, wie tert-Butoxycarbonyl, Arylmethoxycarbonyl mit einem oder zwei Arylresten, die gegebenenfalls, z.B. durch Niederalkyl, z.B. tert-Niederalkyl, wie tert-Butyl, Niederalkoxy, wie Methoxy, Hydroxy, Halogen, wie Chlor, und/oder Nitro, mono- oder polysubstituiertes Phenyl darstellen, z.B. Benzyloxycarbonyl, 4-Nitrobenzyloxycarbonyl, Diphenylmethoxycarbonyl, Di-(4-methoxyphenyl)-methoxycarbonyl oder 9-Fluorenylmethoxycarbonyl, Aroylmethoxycarbonyl, z.B. Phenacyloxycarbonyl, 2-Halogen-niederalkoxycarbonyl, z.B. 2-Chlorethoxycarbonyl, 2,2,2-Trichlorethoxycarbonyl, 2-Bromethoxycarbonyl oder 2-Iodethoxycarbonyl, 2-Triniederalkylsilylniederalkoxycarbonyl, z.B. 2-Trimethlysilylethoxycarbonyl, oder 2-Triarylsilylniederalkoxycarbonyl, z.B. 2-Triphenylsilylethoxycarbonyl.

Eine Arylmethylaminogruppe ist z.B. Mono-, Di- oder insbesondere Triphenylmethylamino, z.B. Benzyl-, Diphenylmethyl- oder Tritylamino. In einer veretherten Mercaptoaminogruppe ist die veretherte Mercaptogruppe in erster Linie substituiertes Arylthio, z.B. 4-Nitrophenylthio. Eine Silylaminogruppe ist beispielsweise eine Triniederalkylsilylaminogruppe, z.B. Trimethylsilylamino.

Bevorzugte Aminoschutzgruppen sind Acylreste von Kohlensäurehalbestern, insbesondere tert-Butoxycarbonyl, gegebenenfalls substituiertes Benzyloxycarbonyl, z.B. 4-Nitrobenzyloxycarbonyl, Diphenylmethoxycarbonyl, 2-Halogen-niederalkoxycarbonyl, z.B. 2,2,2-Trichlorethoxycarbonyl, ferner Trityl und Formyl.

Eine Hydroxygruppe kann beispielsweise durch eine durch Halogen, z.B. Chlor, substituierte Niederalkanoylgruppe, z.B. 2,2-Dichloracetyl, oder insbesondere durch einen für geschützte Aminogruppen genannten Acylrest eines Kohlensäurehalbesters geschützt sein. Eine bevorzugte Hydroxyschutzgruppe ist beispielsweise 2-Chlorethoxycarbonyl, 2,2,2-Trichlorethoxycarbonyl, 4-Nitrobenzyloxycarbonyl oder Diphenylmethoxycarbonyl. Eine Hydroxygruppe kann ferner durch Triniederalkylsilyl, z.B. Trimethylsilyl oder bevorzugt tert-Butyl-dimethylsilyl, eine leicht abspaltbare Alkylgruppe, wie tert-Niederalkyl, z.B. tert-Butyl, einen oxa- oder einen thiaaliphatischen oder -cycloaliphatischen Kohlenwasserstoffrest, beispielsweise 1-Niederalkoxyniederalkyl oder 1-Niederalkylthioniederalkyl, z.B. Methoxymethyl, 1-Methoxyethyl, 1-Ethoxyethyl, Methylthiomethyl, 1-Methylthioethyl oder 1-Ethylthioethyl, oder 2-Oxa- oder 2-Thiacycloalkyl mit 5 - 7 Ringatomen, z.B. 2-Tetrahydrofuryl oder 2-Tetrahydropyranyl, oder ein entsprechendes Thiaanaloges, sowie durch 1-Phenylniederalkyl, z.B. Benzyl, Diphenylmethyl oder Trityl, wobei die Phenylreste beispielsweise durch Halogen, z.B. Chlor, Niederalkoxy, z.B. Methoxy, und/oder Nitro substituiert sein können, geschützt sein.

Die Kondensation zur Herstellung der Amidbindung kann in an sich bekannter Weise durchgeführt werden, beispielsweise wie in Standardwerken, wie "Houben-Weyl, Methoden der organischen Chemie", 4. Auflage, Band 15/II, Georg Thieme Verlag, Stuttgart 1974, "The Peptides" (Herausg. E. Gross und J. Meienhofer), Band 1 und 2, Academic Press, London und New York, 1979/1980, oder M. Bodanszky, "Principles of Peptide Synthesis", Springer-Verlag, Berlin 1984, beschrieben.

Die Kondensation einer freien Carbonsäure mit dem entsprechenden Amin kann in Gegenwart eines der üblichen Kondensationsmittel durchgeführt werden. Uebliche Kondensationsmittel sind z.B. Carbodiimide, beispielsweise Diethyl-, Dipropyl-, N-Ethyl-N'-(3-dimethylaminopropyl)-carbodiimid oder insbesondere Dicyclohexylcarbodiimid, ferner geeignete Carbonylverbindungen, beispielsweise Carbonyldiimidazol, 1,2-Oxazoliumverbindungen, z.B. 2-Ethyl-5-phenyl-1,2-oxazolium-3'-sulfonat und 2-tert-Butyl-5-methylisoxazoliumperchlorat, oder eine geeignete Acylaminoverbindung, z.B. 2-Ethoxy-1-ethoxycarbonyl-1,2-dihydrochinolin, ferner aktivierte Phosphorsäurederivate, z.B. Diphenylphosphorylazid, Diethylphosphorylcyanid, Phenyl-N-phenylphosphoramidochloridat, Bis-(2-oxo-3-oxazolidinyl)-phosphinsäurechlorid oder 1-Benztriazolyloxy-tris-(dimethylamino)-phosphonium-hexafluorophosphat.

Gewünschtenfalls wird eine organische Base zugegeben, z.B. ein Triniederalkylamin mit voluminösen Resten, z.B. Ethyldiisopropylamin, oder eine heterocyclische Base, z.B. Pyridin, 4-Dimethylaminopyridin oder bevorzugt N-Methylmorpholin.

Die Kondensation aktivierter Ester, reaktionsfähiger Anhydride oder reaktionsfähiger cyclischer Amide mit den entsprechenden Aminen wird üblicherweise in Gegenwart einer organischen Base, z.B. einfachen Triniederalkylaminen, z.B. Triethylamin oder Tributylamin, oder einer der vorstehend genannten organischen Basen, durchgeführt. Gewünschtenfalls wird zusätzlich noch ein Kondensationsmittel verwendet, wie für freie Carbonsäuren beschrieben ist.

Die Kondensation von Säureanhydriden mit Aminen kann z.B. in Gegenwart von anorganischen Carbonaten, z.B. Alkalimetallcarbonaten oder -hydrogencarbonaten, wie Natrium- oder Kaliumcarbonat oder -hydrogencarbonat (üblicherweise zusammen mit einem Sulfat), erfolgen.

Carbonsäurechloride, beispielsweise die von der Säure der Formel II abgeleiteten Chlorkohlensäurederivate, werden mit den entsprechenden Aminen vorzugsweise in Gegenwart eines organischen Amins, z.B. der vorstehend genannten Triniederalkylamine oder heterocyclischen Basen, kondensiert.

Die Kondensation wird vorzugsweise in einem inerten, polaren, aprotischen, vorzugsweise wasserfreien, Lösungsmittel oder Lösungsmittelgemisch durchgeführt, beispielsweise in einem Carbonsäureamid, z.B. Formamid oder Dimethylformamid, einem halogenierten Kohlenwasserstoff, z.B. Methylenchlorid, Tetrachlorkohlenstoff oder Chlorbenzol, einem Keton, z.B. Aceton, cyclischen Ether, z.B. Tetrahydrofuran, einem Ester, z.B. Essigsäureethylester, oder einem Nitril, z.B. Acetonitril, oder in Mischungen davon, gegebenenfalls bei erniedrigter oder erhöhter Temperatur, z.B. in einem Temperaturbereich von etwa -40 °C bis etwa +100 °C, bevorzugt von etwa -10 °C bis etwa +500 °C, und gegebenenfalls unter Inertgas-, z.B. Stickstoffatmosphäre.

Reaktionsfähige Säurederivate können auch in situ gebildet werden. So kann man z.B. N,N'-disubstituierte Amidinoester in situ bilden, indem man das Gemisch des Fragments mit freier Carboxygruppe und des komplementären Fragments mit einer Aminogruppe in Gegenwart eines geeigneten disubstituierten Carbodiimids, z.B. Dicyclohexylcarbodiimid, umsetzt. Ferner kann man Amino- oder Amidoester von solchen Säuren in Gegenwart der zu acylierenden Aminokomponente bilden, indem man das Gemisch der entsprechenden Säure- und Amino-Ausgangsstoffe in Gegenwart eines disubstituierten Carbodiimids, z.B. Dicyclohexylcarbodiimid, und eines N-Hydroxylamins oder N-Hydroxyamids, z.B. N-Hydroxybenztriazol, N-Hydroxysuccinimid oder N-Hydroxynorbornan-2,3-dicarbonsäureimid, gegebenenfalls in Anwesenheit einer geeigneten Base, z.B. 4-Dimethylaminopyridin, N-Methylmorpholin oder Ethyldiisopropylamin, umsetzt.

Die Kondensation einer Carbonsäure mit dem entsprechenden, zur Verbindung der Formel I komplementären Fragment mit einer freien Aminogruppe kann auch auf an sich bekannte Weise mit Hilfe von Enzymen erreicht werden, z.B. wie von H.-D. Jakubke et al. in Angewandte Chemie 97,79(1985) beschrieben. Als Enzyme sind beispielsweise Thermolysin, Carboxypeptidase Y, Papain, Chymotrypsin, Trypsin oder Pepsin geeignet. Die Reaktion wird vorzugsweise in Wasser oder in Gemischen von Wasser mit organischen Lösungsmitteln, z.B. mit Niederalkanolen, wie Ethanol, Dimethylformamid, Dimethylsulfoxid, Ethern, wie Tetrahydrofuran, Dioxan oder 1,2-Dimethoxyethan, Aceton, Acetonitril oder Polyalkoholen, z.B. Ethylenglykol, Di-, Tri- oder Poly-ethylenglykol, aber auch mit nicht mischbaren organischen Lösungsmitteln, z.B. Methylenchlorid oder Essigsäureethylester, bei einem pH von 5 bis 8, bevorzugt um den Neutralpunkt, bei Temperaturen zwischen 0 °C und 50 °C durchgeführt. Die Lösungsmittel und die Reaktionsbedingungen werden vorzugsweise so gewählt, dass die gewünschte Verbindung ausfällt oder in die nicht-mischbare organische Phase extrahiert wird und so dem Reaktionsgleichgewicht entzogen wird. Es ist auch möglich, die Kondensation mit auf einem geeigneten Träger immobilisierten Enzymen, wie sie oben genannt sind, in den genannten organischen Lösungsmitteln im Gemisch mit wenig Wasser durchzuführen.

Das Verfahren kann auf an sich bekannte Weise auch in automatisierter Form durchgeführt werden, z.B. gemäss der als Festphasen-Synthese bekannten Technik, die auf R. Merrifield zurückgeht und beispielsweise in Angew. Chem. 97,801-812(1985), Naturwissenschaften 71,252-258 (1984) oder in R.A. Houghten, Proc. Natl. Acad. Sci., 82, 5131-5135(1985) beschrieben ist.

Die Fragmente der Verbindung der Formel I, die gemäss dem genannten Verfahren miteinander kondensiert werden, können auch als Racemate oder als Diastereomerengemische eingesetzt werden. Dabei entstehen Diastereomerengemische, die Verbindungen der Formel I enthalten und noch einer Trennung der Diastereomeren gemäss der unten beschriebenen Nachbehandlung unterworfen werden müssen.

In einer erhältlichen Verbindung der Formel I kann man eine in freier oder in reaktionsfähiger Form vorliegende Carboxygruppe $COOR_2$ verestern bzw. eine veresterte Carboxygruppe $COOR_2$ in eine freie Carboxygruppe überführen.

Zur Veresterung der Carboxygruppe in einer Verbindung der Formel I kann man die freie Säure verwenden oder die freie Säure in eines der oben genannten reaktionsfähigen Derivate überführen und mit einem Alkohol umsetzen, oder man kann die freie Säure oder ein reaktionsfähiges Salz, z.B. das Cäsiumsalz, mit einem reaktionsfähigen Derivat eines Alkohols umsetzen. Beispielsweise kann man das Cäsiumsalz einer Carbonsäure mit dem dem Alkohol entsprechenden Halogenid oder Sulfonsäureester umsetzen. Die Veresterung der Carboxygruppe kann auch mit andern üblichen Alkylierungsmitteln erfolgen, z.B. mit Diazomethan, Meerweinsalzen oder 1-substituierten 3-Aryltriazenen.

Zur Ueberführung einer veresterten Carboxygruppe einer Verbindung der Formel 1 in eine freie Carboxygruppe kann eine der weiter unten bei der Abspaltung der Carboxyschutzgruppen beschriebenen Methoden oder gewünschtenfalls eine alkalische Verseifung nach den im Organikum, 17. Auflage, VEB Deutscher Verlag der Wissenschaften, Berlin (Ost) 1988, genannten Reaktionsbedingungen angewendet werden.

In einer Verbindung der Formel I kann man eine veresterte Carboxygruppe durch Aminolyse mit Ammoniak oder einem Niederalkylamin in eine Carboxamidgruppe der Formel $-CO-NHR_2$ überführen. Die Aminolyse kann nach den im Organikum, 15. Auflage, VEB Deutscher Verlag der Wissenschaften, Berlin

(Ost) 1976, für solche Umsetzungen genannten Reaktionsbedingungen erfolgen oder vorzugsweise durch Umsetzung in einem inerten Lösungsmittel oder insbesondere ohne Zugabe eines Lösungsmittels, bei Normaldruck oder erhöhtem Druck, beispielsweise in einem Bombenrohr, bei Temperaturen von 0° bis 100° C, bevorzugt von 20° bis 80° C°.

In einer erhältlichen Verbindung der Formel I kann man die Thiogruppe von Thiomorpholino $R_1$ zu einer Sulfonylgruppe oxidieren.

Die Oxidation zur Sulfonylgruppe kann mit den meisten der üblichen Oxidationsmittel durchgeführt werden. Bevorzugt verwendet man solche Oxidationsmittel, die die Thiogruppe selektiv in Gegenwart anderer funktioneller Gruppen der Verbindung der Formel I, z.B. der Amidfunktion und der Hydroxygruppe, oxidieren, beispielsweise aromatische oder aliphatische Peroxycarbonsäuren, z.B. Perbenzoesäure, Monoperphthalsäure, m-Chlorperbenzoesäure, Peressigsäure, Perameisensäure oder Trifluorperessigsäure. Die Oxidation mit Peroxycarbonsäuren erfolgt in den üblichen dafür geeigneten Lösungsmitteln, beispielsweise Chlorkohlenwasserstoffen, z.B. Methylenchlorid oder Chloroform, Diethylether oder Essigester oder dergleichen, bei Temperaturen zwischen -78° C und Raumtemperatur, z.B. zwischen -20° C und +10° C, bevorzugt um 0° C. Die Peroxycarbonsäure kann auch in situ gebildet werden, z.B. mit Wasserstoffperoxid in Essigsäure oder Ameisensäure, die gegebenenfalls Essigsäureanhydrid enthält, z.B. mit 30 % oder 90 % Wasserstoffperoxid in Essigsäure/Essigsäureanhydrid. Geeignet sind auch andere Peroxoverbindungen, beispielsweise Kaliumperoxomonosulfat in Niederalkanol/Wasser-Mischungen, z.B. Methanol-Wasser oder Ethanol-Wasser, oder in wässriger Essigsäure bei Temperaturen zwischen -70° C und +30° C, z.B. zwischen -20° C und Raumtemperatur, ferner Natriummetaperiodat in Methanol oder Methanol-Wasser-Gemischen bei Temperaturen zwischen 0° C und 50° C, z.B. um Raumtemperatur.

Gewünschtenfalls kann in einer erhältlichen Verbindung der Formel I die Sulfonylgruppe von S,S-Dioxothiomorpholino $R_1$ zu einer Thiogruppe reduziert werden, beispielsweise mit Diisobutylaluminiumhydrid in Diethylether oder Tetrahydrofuran.

In einer erhältlichen Verbindung der Formel I, worin eine oder mehrere funktionelle Gruppen geschützt sind, können diese Gruppen, z.B. Carboxy-, Amino- oder Hydroxygruppen, in an sich bekannter Weise, mittels Solvolyse, insbesondere Hydrolyse, gegebenenfalls enzymatischer Hydrolyse, Alkoholyse oder Acidolyse, oder mittels Reduktion, insbesondere Hydrogenolyse, oder chemischer Reduktion, gegebenenfalls stufenweise oder gleichzeitig, freigesetzt werden. Die Abspaltung der Schutzgruppen ist in den weiter vorn im Abschnitt "Schutzgruppen" genannten Standardwerken beschrieben. Beispielsweise kann man geschütztes Carboxy, z.B. tert-Niederalkoxycarbonyl, in 2-Stellung durch eine organische Silylgruppe oder in 1-Stellung durch Niederalkoxy oder Niederalkylthio substituiertes Niederalkoxycarbonyl oder gegebenenfalls substituiertes Diphenylmethoxycarbonyl, durch Behandeln mit einer geeigneten Säure, z.B. Ameisensäure oder Trifluoressigsäure, gegebenenfalls unter Zugabe einer nukleophilen Verbindung, z.B. Phenol oder Anisol, in freies Carboxy überführen. Gegebenenfalls substituiertes Benzyloxycarbonyl kann z.B. mittels Hydrogenolyse, d.h. durch Behandeln mit Wasserstoff in Gegenwart eines metallischen Hydrierkatalysators, wie eines Palladiumkatalysators, freigesetzt werden. Ferner kann man geeignet substituiertes Benzyloxycarbonyl, wie 4-Nitrobenzyloxycarbonyl, auch durch Reduktion, z.B. durch Behandeln mit einem Alkalimetalldithionit, z.B. Natrium-dithionit, mit einem reduzierenden Metall, z.B. Zink, oder einem reduzierenden Metallsalz, wie einem Chrom(II)-salz, z.B. Chrom(II)-chlorid, üblicherweise in Gegenwart eines Wasserstoffabgebenden Mittels, das zusammen mit dem Metall naszierenden Wasserstoff zu erzeugen vermag, wie einer Säure, in erster Linie einer geeigneten Carbonsäure, wie einer gegebenenfalls, z.B. durch Hydroxy, substituierten Niederalkancarbonsäure, z.B. Essigsäure, Ameisensäure, Glycolsäure, Diphenylglycolsäure, Milchsäure, Mandelsäure, 4-Chlormandelsäure oder Weinsäure, oder eines Alkohols oder Thiols, wobei man vorzugsweise Wasser zugibt, in freies Carboxy überführen. Durch Behandeln mit einem reduzierenden Metall oder Metallsalz, wie oben beschrieben, kann man auch 2-Halogenniederalkoxycarbonyl (gegebenenfalls nach Umwandlung einer 2-Bromniederalkoxycarbonylgruppe in eine entsprechende 2-Iodniederalkoxycarbonylgruppe) oder Aroylmethoxycarbonyl in freies Carboxy umwandeln. Aroylmethoxycarbonyl kann ebenfalls durch Behandeln mit einem nukleophilen, vorzugsweise salzbildenden Reagens, wie Natriumthiophenolat oder Natriumiodid, gespalten werden. 2-Triniederalkylsilylniederalkoxycarbonyl kann auch durch Behandeln mit einem das Fluoridanion liefernden Salz der Fluorwasserstoffsäure, wie einem Alkalimetallfluorid, z.B. Natrium- oder Kaliumfluorid, gegebenenfalls in Anwesenheit eines makrozyclischen Polyethers ("Kronenether"), oder mit einem Fluorid einer organischen quaternären Base, wie Tetraniederalkylammoniumfluorid oder Triniederalkyl-arylniederalkylammoniumfluorid, z.B. Tetraethylammoniumfluorid oder Tetrabutylammoniumfluorid, in Gegenwart eines aprotischen, polaren Lösungsmittels, wie Dimethylsulfoxid oder N,N-Dimethylacetamid, in freies Carboxy übergeführt werden. Mit einer organischen Silylgruppe, wie Triniederalkylsilyl, z.B. Trimethylsilyl, verestertes Carboxy kann in üblicher Weise solvolytisch, z.B. durch Behandeln mit Wasser, einem Alkohol oder Säure, oder ausserdem einem Fluorid, wie

oben beschrieben, freigesetzt werden. Verestertes Carboxy kann auch enzymatisch gespalten werden, z.B. verestertes Arginin oder Lysin, wie Lysin-methylester, mittels Trypsin.

Eine geschützte Aminogruppe setzt man in an sich bekannter und je nach Art der Schutzgruppen in verschiedenartiger Weise, vorzugsweise mittels Solvolyse oder Reduktion, frei. 2-Halogenniederalkoxycarbonylamino (gegebenenfalls nach Umwandlung einer 2-Bromniederalkoxycarbonylaminogruppe in eine 2-Iodniederalkoxycarbonylaminogruppe), Aroylmethoxycarbonylamino oder 4-Nitrobenzyloxycarbonylamino kann z.B. durch Behandeln mit einem geeigneten Reduktionsmittel, wie Zink in Gegenwart einer geeigneten Carbonsäure, wie wässriger Essigsäure, gespalten werden. Aroylmethoxycarbonylamino kann auch durch Behandeln mit einem nukleophilen, vorzugsweise salzbildenden Reagens, wie Natriumthiophenolat, und 4-Nitrobenzyloxycarbonylamino auch durch Behandeln mit einem Alkalimetall-, z.B. Natrium-dithionit, gespalten werden. Gegebenenfalls substituiertes Diphenylmethoxycarbonylamino, tert-Niederalkoxycarbonylamino oder 2-Triniederalkylsilylniederalkoxycarbonylamino kann durch Behandeln mit einer geeigneten Säure, z.B. Ameisen- oder Trifluoressigsäure, gegebenenfalls substituiertes Benzyloxycarbonylamino z.B. mittels Hydrogenolyse, d.h. durch Behandeln mit Wasserstoff in Gegenwart eines geeigneten Hydrierkatalysators, wie eines Palladiumkatalysators, gegebenenfalls substituiertes Triarylmethylamino oder Formylamino z.B. durch Behandeln mit einer Säure, wie Mineralsäure, z.B. Chlorwasserstoffsäure, oder einer organischen Säure, z.B. Ameisen-, Essig- oder Trifluoressigsäure, gegebenenfalls in Gegenwart von Wasser, und eine mit einer organischen Silylgruppe geschützte Aminogruppe z.B. mittels Hydrolyse oder Alkoholyse freigesetzt werden. Eine durch 2-Halogenacetyl, z.B. 2-Chloracetyl, geschützte Aminogruppe kann durch Behandeln mit Thioharnstoff in Gegenwart einer Base, oder mit einem Thiolatsalz, wie einem Alkalimetallthiolat des Thioharnstoffs, und anschliessende Solvolyse, wie Alkoholyse oder Hydrolyse, des entstandenen Substitutionsprodukts freigesetzt werden. Eine durch 2-Triniederalkylsilylniederalkoxycarbonyl geschützte Aminogruppe kann auch durch Behandeln mit einem Fluoridanionen liefernden Salz der Fluorwasserstoffsäure, wie oben im Zusammenhang mit der Freisetzung einer entsprechend geschützten Carboxygruppe angegeben, in die freie Aminogruppe überführt werden. Ebenso kann man direkt an ein Heteroatom, wie Stickstoff, gebundenes Silyl, wie Trimethylsilyl, mittels Fluoridionen abspalten.

Eine durch eine geeignete Acylgruppe, eine organische Silylgruppe oder durch gegebenenfalls substituiertes 1-Phenylniederalkyl geschützte Hydroxygruppe wird analog einer entsprechend geschützten Aminogruppe freigesetzt. Eine durch 2,2-Dichloracetyl geschützte Hydroxygruppe wird z.B. durch basische Hydrolyse, eine durch tert-Niederalkyl oder durch einen 2-oxa- oder 2-thia-aliphatischen oder -cycloaliphatischen Kohlenwasserstoffrest geschützte Gruppe durch Acidolyse, z.B. durch Behandeln mit einer Mineralsäure oder einer starken Carbonsäure, z.B. Trifluoressigsäure, freigesetzt. Eine Silylgruppe, z.B. eine Trimethylsilyl- oder tert-Butyldimethylsilylgruppe, wird ebenfalls durch Acidolyse, z.B. durch Mineralsäure, bevorzugt Fluorwasserstoffsäure, oder eine starke Carbonsäure abgespalten. 2-Halogenniederalkoxycarbonyl wird durch die obengenannten Reduktionsmittel, z.B. reduzierendes Metall, wie Zink, reduzierende Metallsalze, wie Chrom(II)-salze, oder durch Schwefelverbindungen, beispielsweise Natriumdithionit oder bevorzugt Natriumsulfid und Schwefelkohlenstoff, entfernt.

Salze von Verbindungen der Formel I mit salzbildenden Gruppen können in an sich bekannter Weise hergestellt werden. So kann man Salze von Verbindungen der Formel I mit sauren Gruppen z.B. durch Behandeln mit Metallverbindungen, wie Alkalimetallsalzen von geeigneten organischen Carbonsäuren, z.B. dem Natriumsalz der 2-Ethylhexansäure, oder mit anorganischen Alkali- oder Erdalkalimetallsalzen, z.B. Natriumhydrogencarbonat, oder mit Ammoniak oder einem geeigneten organischen Amin bilden, wobei man vorzugsweise stöchiometrische Mengen oder nur einen kleinen Ueberschuss des salzbildenden Mittels verwendet. Säureadditionssalze von Verbindungen der Formel I erhält man in üblicher Weise, z.B. durch Behandeln mit einer Säure oder einem geeigneten Anionenaustauscherreagens. Innere Salze von Verbindungen der Formel I, welche z.B. eine freie Carboxygruppe und eine freie Aminogruppe enthalten, können z.B. durch Neutralisieren von Salzen, wie Säureadditionssalzen, auf den isoelektrischen Punkt, z.B. mit schwachen Basen, oder durch Behandeln mit Ionenaustauschern gebildet werden.

Salze können in üblicher Weise in die freien Verbindungen übergeführt werden: Metall- und Ammoniumsalze z.B. durch Behandeln mit geeigneten Säuren, Säureadditionssalze z.B. durch Behandeln mit einem geeigneten basischen Mittel.

Stereoisomerengemische, insbesondere Diastereomerengemische, können in an sich bekannter Weise, z.B. durch fraktionierte Kristallisation, Chromatographie etc., in die einzelnen Isomeren aufgetrennt werden.

Racemate können in an sich bekannter Weise, z.B. nach Ueberführung der optischen Antipoden in Diastereomere, beispielsweise durch Umsetzung mit optisch aktiven Säuren oder Basen, gespalten werden.

Die Erfindung betrifft auch diejenigen Ausführungsformen des Verfahrens, bei denen man von einer auf irgendeiner Stufe als Zwischenprodukt erhältlichen Verbindung ausgeht und die fehlenden Schritte durchführt oder das Verfahren auf irgendeiner Stufe abbricht oder eine nach dem erfindungsgemässen Verfahren

erhältliche Verbindung unter den Verfahrensbedingungen erzeugt und in situ weiterverarbeitet.

Pharmazeutische Präparate:

Die pharmakologisch verwendbaren Verbindungen der vorliegenden Erfindung, insbesondere die nach den vorstehenden Verfahren erhaltenen Verbindungen der Formel I, können z.B. zur Herstellung von pharmazeutischen Präparaten verwendet werden, welche eine wirksame Menge des Wirkstoffs zusammen oder im Gemisch mit einer signifikanten Menge von anorganischen oder organischen, festen oder flüssigen, pharmazeutisch verwendbaren Trägerstoffen enthalten.

Bei den erfindungsgemässen pharmazeutischen Präparaten handelt es sich um solche zur enteralen, wie nasalen, rektalen oder oralen, oder parenteralen, wie intramuskulären oder intravenösen, Verabreichung an Warmblüter (Menschen und Tiere), welche eine effektive Dosis des pharmakologischen Wirkstoffs allein oder zusammen mit einer signifikanten Menge eines pharmazeutisch anwendbaren Trägermaterials enthalten. Die Dosierung des Wirkstoffs hängt von der Warmblüter-Spezies, dem Körpergewicht, Alter und dem individuellen Zustand, der zu behandelnden Krankheit sowie von der Applikationsweise ab.

Die Erfindung betrifft auch eine Methode zur Behandlung von durch Retroviren verursachten Krankheiten, z.B. von AIDS, dadurch gekennzeichnet, dass eine therapeutisch hiergegen wirksame Menge von erfindungsgemässen Verbindungen der Formel I verabreicht wird. Die an Warmblüter, insbesondere an unter durch Retroviren verursachten Erkrankungen leidende, z.B. Menschen von etwa 70 kg Körpergewicht, zu verabreichenden Dosismengen liegen zwischen etwa 3 mg und etwa 3 g, vorzugsweise zwischen etwa 10 mg und etwa 1,5 g, z.B. bei ungefähr 300 mg bis 1000 mg pro Person und Tag, verteilt auf vorzugsweise 1 bis 3 Einzeldosen, die z.B. gleich gross sein können. Ueblicherweise erhalten Kinder die halbe Dosis von Erwachsenen.

Die neuen pharmazeutischen Präparate enthalten von etwa 1 % bis etwa 95 %, vorzugsweise von etwa 20 % bis etwa 90 % des Wirkstoffes. Erfindungsgemässe pharmazeutische Präparate können z.B. in Dosiseinheitsform, wie Ampullen, Vials, Suppositorien, Dragées, Tabletten oder Kapseln, vorliegen.

Die pharmazeutischen Präparate der vorliegenden Erfindung werden in an sich bekannter Weise, z.B. mittels konventioneller Lösungs-, Lyophilisierungs-, Misch-, Granulier- oder Dragierverfahren, hergestellt.

Vorzugsweise verwendet man Lösungen des Wirkstoffs, daneben auch Suspensionen, und zwar insbesondere isotonische wässrige Lösungen oder Suspensionen, wobei diese z.B. bei lyophilisierten Präparaten, welche die Wirksubstanz allein oder zusammen mit einem Trägermaterial, z.B. Mannit, enthalten, vor Gebrauch hergestellt werden können. Die pharmazeutischen Präparate können sterilisiert sein und/oder Hilfsstoffe, z.B. Konservier-, Stabilisier-, Netz- und/oder Emulgiermittel, Löslichkeitsvermittler, Salze zur Regulierung des osmotischen Druckes und/oder Puffer enthalten und werden in an sich bekannter Weise, z.B. mittels konventioneller Lösungs- oder Lyophilisierungsverfahren, hergestellt. Die genannten Lösungen oder Suspensionen können viskositätserhöhende Stoffe, wie Natriumcarboxymethylcellulose, Carboxymethylcellulose, Dextran, Polyvinylpyrrolidon oder Gelatine, enthalten.

Suspensionen in Oel enthalten als ölige Komponente die für Injektionszwecke gebräuchlichen vegetabilen, synthetischen oder halbsynthetischen Oele. Als solche sind insbesondere flüssige Fettsäureester zu nennen, die als Säurekomponente eine langkettige Fettsäure mit 8 - 22, besonders 12 - 22, Kohlenstoffatomen, wie z.B. Laurinsäure, Tridecylsäure, Myristinsäure, Pentadecylsäure, Palmitinsäure, Margarinsäure, Stearinsäure, Arachinsäure, Behensäure oder entsprechende ungesättigte Säuren wie z.B. Oelsäure, Elaidinsäure, Erucasäure, Brasidinsäure oder Linolsäure, enthalten. Die Alkoholkomponente dieser Fettsäureester hat maximal 6 Kohlenstoffatome und ist ein ein- oder mehrwertiger, z.B. ein-, zwei- oder dreiwertiger Alkohol, z.B. Methanol, Ethanol, Propanol, Butanol oder Pentanol oder deren Isomere, vor allem aber Glycol oder Glycerin. Als Fettsäureester sind daher beispielsweise zu nennen: Ethyloleat, Isopropylmyristat, Isopropylpalmitat, "Labrafil M 2735" (Polyoxyethylenglycerintrioleat der Firma Gattefossé, Paris), "Myglyol 812" (Triglycerid gesättigter Fettsäuren der Kettenlänge $C_8$ bis $C_{12}$ der Firma Chemische Werke Witten/Ruhr, Deutschland), besonders aber vegetabile Oele wie Baumwollsaatöl, Mandelöl, Olivenöl, Ricinusöl, Sesamöl, Sojabohnenöl und vor allem Erdnussöl.

Die Herstellung der Injektionspräparate erfolgt in üblicher Weise unter sterilen Bedingungen, ebenso das Abfüllen in Ampullen oder Vialen sowie das Verschliessen der Behälter.

Pharmazeutische Präparate zur oralen Anwendung können erhalten werden, indem man den Wirkstoff mit festen Trägerstoffen kombiniert, ein erhaltenes Gemisch gegebenenfalls granuliert und das Gemisch bzw. Granulat, wenn erwünscht oder notwendig nach Zugabe von geeigneten Hilfsstoffen, zu Tabletten oder Dragée-Kernen verarbeitet. Dabei kann man sie auch in Kunststoffträger einbauen, die die Wirkstoffe dosiert abgeben oder diffundieren lassen.

Geeignete Trägerstoffe sind insbesondere Füllstoffe, wie Zucker, z.B. Lactose, Saccharose, Mannit oder

Sorbit, Cellulosepräparate und/oder Calciumphosphate, z.B. Tricalciumphosphat oder Calciumhydrogenphosphat, ferner Bindemittel, wie Stärkekleister unter Verwendung z.B. von Mais-, Weizen-, Reis- oder Kartoffelstärke, Gelatine, Traganth, Methylcellulose, Hydroxypropylmethylcellulose, Natriumcarboxymethylcellulose und/oder Polyvinylpyrrolidon, und/oder, wenn erwünscht, Sprengmittel, wie die obengenannten Stärken, ferner Carboxymethylstärke, quervernetztes Polyvinylpyrrolidon, Agar, Alginsäure oder ein Salz davon, wie Natriumalginat. Hilfsmittel sind in erster Linie Fliessregulier- und Schmiermittel, z.B. Kieselsäure, Talk, Stearinsäure oder Salze davon, wie Magnesium- oder Calciumstearat, und/oder Polyethylenglykol. Dragée-Kerne werden mit geeigneten, gegebenenfalls magensaftresistenten Ueberzügen versehen, wobei man u.a. konzentrierte Zuckerlösungen, welche gegebenenfalls arabischen Gummi, Talk, Polyvinylpyrrolidon, Polyethylenglykol und/oder Titandioxid enthalten, Lacklösungen in geeigneten organischen Lösungsmitteln oder Lösungsmittelgemischen oder, zur Herstellung von magensaftresistenten Ueberzügen, Lösungen von geeigneten Cellulosepräparaten, wie Ethylcellulosephthalat oder Hydroxypropylmethylcellulosephthalat, verwendet. Den Tabletten oder Dragée-Ueberzügen können Farbstoffe oder Pigmente, z.B. zur Identifizierung oder zur Kennzeichnung verschiedener Wirkstoffdosen, beigefügt werden.

Ausgangsmaterialien:

Vorzugsweise werden solche Ausgangsstoffe verwendet und die Reaktionsbedingungen so gewählt, dass man zu den als bevorzugt aufgeführten Verbindungen gelangt.

Die Ausgangsmaterialien zur Durchführung des oben genannten Verfahrens sind bekannt oder können nach an sich bekannten Verfahren hergestellt werden, z.B. aus den betreffenden Aminosäuren durch Kondensation analog dem vorstehend beschriebenen Verfahren. Beispielsweise kann man eine Verbindung der Formel IV, V, VI, VII oder VIII analog dem in den Europäischen Patentanmeldungen EP 143 746 (publiziert am 05.06.1985) oder EP 258 183 (publiziert am 02.03.1988) beschriebenen Verfahren herstellen.

Die folgenden Beispiele dienen zur Illustration der Erfindung, schränken deren Umfang jedoch in keiner Weise ein.

Temperaturen werden in Celsiusgraden angegeben. Die $R_f$-Werte werden auf Kieselgeldünnschichtplatten in folgenden Lösungsmittelsystemen ermittelt:

| | | |
|---|---|---|
| A | Essigester-n-Hexan | 1:1 |
| B | Essigester-n-Hexan | 1:2 |
| C | Essigester-n-Hexan | 1:4 |
| D | Essigester-n-Hexan | 1:6 |
| E | Essigester-n-Hexan | 1:9 |
| F | Methylenchlorid-Methanol | 19:1 |
| G | Methylenchlorid-Methanol | 9:1 |
| H | Methylenchlorid-Methanol | 4:1 |
| I | Methylenchlorid-Methanol-Wasser | 300:10:1 |
| J | Methylenchlorid-Diethylether | 4:1 |
| K | Methylenchlorid-Methanol-Wasser | 14:6:1 |
| L | Chloroform-Methanol-Wasser-Eisessig | 170:26:3:1 |

Beispielsweise bedeutet die Abkürzung "$R_f$(A)", dass der $R_f$-Wert im System A ermittelt wurde. Das Mengenverhältnis der Lösungsmittel zueinander ist in Volumenanteilen angegeben.

Die gleichen Abkürzungen werden für die Bezeichnung der Fliessmittel-Systeme bei der Flash-Chromatographie und der Mitteldruckchromatographie verwendet.

Die Werte für Infrarot-Spektroskopie (IR) werden in $cm^{-1}$ angegeben. Die Werte für Protonen-Kernresonanzspektroskopie ($^1H$-NMR) werden in ppm (parts per million) bezogen auf Tetramethylsilan als internen Standard angegeben. s = Singulett, d = Dublett, t = Triplett, q = Quartett, m = Multiplett, dd = Doppeldublett, br = breit. Bei der "fast atom bombardment" Massenspektrometrie (FAB-MS) sind die Werte für die protonierte Masse $(M+H)^+$ angegeben.

Der Rest mit der Bezeichnung

-Cha$^{\underline{c}}$Val-

bedeutet das zweiwertige Radikal von (2S,4S,5S)-5-Amino-6-cyclohexyl-4-hydroxy-2-isopropyl-hexansäure und hat die Formel

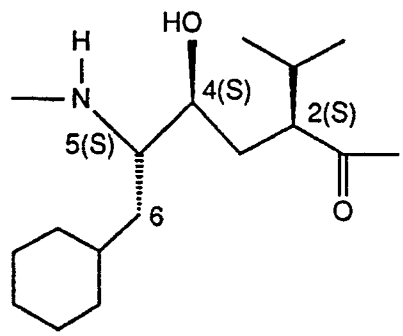

Der Rest mit der Bezeichnung -Cha$^{\underline{cx}}$Val- leitet sich vom Rest

-Cha$^{\underline{c}}$Val-

durch Ueberbrückung von NH und OH durch eine Isopropyliden-Gruppe ab und hat die Formel

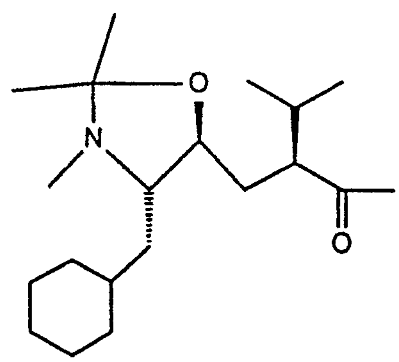

Zur Bezeichnung von zweiwertigen Radikalen von natürlichen $\alpha$-Aminosäuren werden die in der Peptidchemie üblichen Abkürzungen verwendet. An der phenolischen Hydroxygruppe mit dem Rest R veresterte oder veretherte Tyrosin-Radikale werden mit Tyr(R) bezeichnet.

Weitere Abkürzungen:

| | |
|---|---|
| abs. | = absolut (wasserfrei) |
| DCCI | = Dicyclohexylcarbodiimid |
| DCH | = Dicyclohexylharnstoff |
| DMF | = Dimethylformamid |
| DMSO | = Dimethylsulfoxid |
| Ether | = Diethylether |
| Fmoc | = 9-Fluorenylmethoxycarbonyl |
| HOBt | = 1-Hydroxybenztriazol |
| Me | = Methyl |
| Min. | = Minute(n) |
| Smp. | = Schmelzpunkt |
| Std. | = Stunde(n) |

Tcp = 2,4,5-Trichlorphenyl
THF = Tetrahydrofuran
Z = Benzyloxycarbonyl

Beispiel 1:

S,S-Dioxothiomorpholinocarbonyl-Val-Cha $\overset{C}{-}$ Val-Val-Tyr-OMe

Zu 45 mg (0,069 mMol)

H-Val-Cha $\overset{C}{-}$ Val-Val-Tyr-OMe

gelöst in 2 ml DMF werden bei bei Raumtemperatur 34,8 µl Triethylamin und dann 16,6 mg (0,083 mMol) S,S-Dioxothiomorpholinocarbonylchlorid in 2 ml Methylenchlorid zugegeben. Die Mischung wird 2 Std. bei Raumtemperatur gerührt, eingeengt und der Rückstand in wenig Diisopropylether digeriert. Das Rohprodukt wird durch Chromatographie an Kieselgel (Laufmittel G) gereinigt. $R_f$(L) = 0,42; FAB-MS:: (M+H)+ = 808.

Die Ausgangsmaterialien werden folgendermassen hergestellt:

a) 2(S)-Benzyloxycarbonylamino-3-cyclohexyl-propionsäureethylester: 243 g 2(S)-Benzyloxycarbonylamino-3-cyclohexyl-propionsäure (Herstellung: Helvetica Chimica Acta 57, 2131-(1974)) werden in 600 ml Toluol und 900 ml Ethanol vorgelegt. Das Reaktionsgemisch wird auf 0° gekühlt und 88,3 g Thionylchlorid innerhalb 30 Min. zugetropft. Die Kühlung wird entfernt und die Mischung während 18 Std. gerührt. Das Reaktionsgemisch wird filtriert und das Filtrat eingeengt. Der Rückstand wird mittels Flash-Chromatographie (2 kg Kieselgel 60, 40-63 µm, Laufmittel E) aufgetrennt. Die produkthaltigen Fraktionen werden vereinigt, eingedampft und im Hochvakuum getrocknet. Man erhält die Titelverbindung als leicht gelbliches Oel. $R_f$ (E) = 0,2; $R_f$ (B) = 0,52.

b) 2(S)-Benzyloxycarbonylamino-3-cyclohexyl-propanal: 116,1 g 2(S)-Benzyloxycarbonylamino-3-cyclohexyl-propionsäureethylester werden in 2,2 l Toluol vorgelegt und auf -65° abgekühlt. 836 ml Diisobutylaluminiumhydrid werden innerhalb 30 Min. tropfenweise bei -65° zugegeben und das Gemisch 20 Min. nachgerührt. Dann werden bei -65° 84,2 ml Methanol innerhalb 10 Min. zugetropft, anschliessend 825 ml wässrige Kaliumnatrium-tartrat-Lösung ohne Kühlung. Das Reaktionsgemisch wird auf 3 l Kaliumnatrium-tartrat-Lösung/Eis ausgetragen und mit 5 l Ether extrahiert. Die Etherphase wird mit 2 l Wasser gewaschen, dann sofort in eine Lösung bestehend aus 106 g Semicarbazid-Hydrochlorid und 156,5 g Natriumacetat in 620 ml Wasser und 620 ml Ethanol gegossen. Das Reaktionsgemisch wird 1 Std. bei Raumtemperatur nach gerührt, dann im Scheidetrichter abgetrennt und die Wasserphase mit 2 x 1,5 l Ether extrahiert. Die organische Phase wird über Magnesiumsulfat getrocknet und eingedampft. Das Rohprodukt wird mittels Flash-Chromatographie gereinigt (2 kg Kieselgel 60, 40-63 µm, Laufmittel A). Durch Eindampfen der vereinigten, produkthaltigen Fraktionen erhält man das Semicarbazon der Titelverbindung, $R_f$ (G) = 0,51. 130 g dieses Semicarbazons werden in 1 l THF gelöst, mit 282 ml 37 % Formaldehydlösung und dann bei 10° mit 143 ml 0,5N HCl versetzt. Das Reaktionsgemisch wird 2 Std. bei Raumtemperatur gerührt, filtriert und das Filtrat mit 0,5 l Wasser, 0,5 l $NaHCO_3$ und 0,5 l Wasser gewaschen. Die Wasserphasen werden mit 600 ml Ether extrahiert. Die Etherphasen werden über Magnesiumsulfat getrocknet und eingedampft. Der Rückstand wird mit 100 ml Toluol versetzt und eingedampft, wobei die Titelverbindung erhalten wird. Diese wird sofort weiterverarbeitet.

c) (1(S)-Benzyloxycarbonylamino-2-cyclohexyl-ethyl)-oxiran: 18,9 g Natriumhydrid-dispersion (55 % in Oel) werden in einem trockenen Sulfierkolben unter Argon durch dreimaliges Aufrühren in 50 ml Petrolether (Sdp. 40-60°) und anschliessendes Abdekantieren des Lösungsmittels vom Oel befreit. Nach Trocknen im Hochvakuum wird ein graues Pulver erhalten, das in 500 ml THF vorgelegt und mit 55,6 g Trimethylsulfoxoniumiodid versetzt wird, wobei die Temperatur auf ca. 40° steigt. Die graue Suspension wird am Rückfluss 1 Std. gekocht und anschliessend innerhalb von 50 Min. bei -70° mit einer Lösung von 108,6 g 2(S)-Benzyloxycarbonyl-amino-3-cyclohexylpropanal in 250 ml THF versetzt. Die gelbe Suspension wird 2 Std. bei 0° gerührt. Die gelblich-trübe Lösung wird auf 500 g Eis gegossen. Die wässrige Lösung wird mit 2,5 l Ether extrahiert, die organische Phase mit Wasser gewaschen und nach Trocknen über Natriumsulfat eingedampft. Der ölige Rückstand wird mittels Flash-Chromatographie (2,5 kg Kieselgel 60, 40-63 µm, Laufmittel C) aufgetrennt. Die produkthaltigen Fraktionen werden vereinigt, eingedampft und im Hochvakuum getrocknet. Man erhält die Titelverbindung (Diastereomerengemisch,

ca. 4:1) als leicht gelbliches Oel. $R_f$(I) = 0,71; $R_f$(C) = 0,16.

d) 3(S)-Benzyloxycarbonylamino-4-cyclohexyl-1-iod-butan-2(R,S)-ol: 42,3 g (1(S)-Benzyloxycarbonylamino-2-cyclohexyl-ethyl)-oxiran werden in 200 ml Acetonitril aufgenommen und die erhaltene Lösung auf 0° abgekühlt. Nach Zugabe von 20,9 g Natriumiodid werden während 30 Min. tropfenweise bei 0° 17,7 ml Trimethylchlorsilan zugegeben. Das Gemisch wird 40 Min. bei 0 - 3° gerührt und anschliessend auf 700 ml eiskaltes Wasser gegossen. Das wässrige Gemisch wird mit Ether extrahiert und die organische Phase mit 750 ml 5 %-iger wässriger Natriumthiosulfatlösung und 750 ml gesättigter, wässriger Natriumchloridlösung gewaschen. Nach Trocknen über Natriumsulfat und Eindampfen erhält man ein öliges Gemisch der Titelverbindung, die direkt weiterverarbeitet wird.

e) 3-Benzyloxycarbonyl-4(S)-cyclohexylmethyl-2,2-dimethyl-5(R)-iodmethyl-1,3-oxazolidin: 49,3 g der Verbindung Beispiel 1d) und 1,07 g p-Toluolsulfonsäuremonohydrat werden in 140 ml 2,2-Dimethoxypropan und 450 ml Methylenchlorid 3 Std. lang bei Raumtemperatur gerührt. Das Gemisch wird zwischen 1 l Methylenchlorid und 500 ml gesättigter, wässriger Natriumhydrogencarbonatlösung ausgeschüttelt. Die organische Phase wird mit Wasser gewaschen, über Natriumsulfat getrocknet und eingedampft. Das Rohprodukt wird mittels Flash-Chromatographie gereinigt (3 kg Kieselgel 60, 40-63 µm, Laufmittel D). Durch Eindampfen der vereinigten produkthaltigen Fraktionen erhält man die Titelverbindung als leicht gelbliches Oel. $R_f$ (c) = 0,55; $R_f$ (D) = 0,46.

f) 2(R,S)-(3-Benzyloxycarbonyl-4(S)-cyclohexylmethyl-2,2-dimethyl-1,3-oxazolidinyl-5(S)-methyl)-3-methyl-buttersäuremethylester: 14,3 ml Diisopropylamin werden in 200 ml abs. THF unter Argon gelöst und auf 0° abgekühlt. Anschliessend wird bei 0 - 5° das Gemisch 20 Min. lang tropfenweise mit 65,8 ml einer 1,6 M Lösung von n-Butyl-lithium in Hexan versetzt und 20 Min. gerührt. Dann werden bei -70° bis -75° 13,3 ml Isovaleriansäuremethylester zugetropft und die Mischung 1,5 Std. bei -75° gerührt. Bei -60° bis -75° werden unter Rühren 320 ml Hexamethylphosphorsäuretriamid zugetropft. Die entstandene Suspension wird 10 Min. lang gerührt und schliesslich bei -70° bis -75° innerhalb von 5 Min. tropfenweise mit einer Lösung von 43,4 g der Verbindung Beispiel 1e) in 110 ml Tetrahydrofuran versetzt. Das Reaktionsgemisch wird bei Raumtemperatur während 2,5 Std. gerührt und schliesslich auf ein Gemisch von 1 l gesättigter, wässriger Ammoniumchloridlösung und 500 g Eis gegossen. Die wässrige Phase wird mit 2 l Essigester extrahiert, die organische Phase mit Wasser gewaschen und über Natriumsulfat getrocknet. Nach Eindampfen erhält man das Diastereomerengemisch der Titelverbindung als gelbes Oel. $R_f$ (C) = 0,36; $R_f$ (E) = 0,21 (Werte für die weniger polare Komponente).

g) 2(R,S)-(3-Benzyloxycarbonyl-4(S)-cyclohexylmethyl-2,2-dimethyl-1,3-oxazolidinyl-5(S)-methyl)-3-methyl-buttersäure: 16,5 g Kalium-tert-butylat werden in 250 ml Ether bei ca. 5° mit 1,77 ml Wasser versetzt. Die weisse Suspension wird noch 10 Min. im Eisbad gerührt und darauf mit 35,8 g der Verbindung Beispiel 1f) (Diastereomerengemisch) in 250 ml Ether versetzt, wobei die Temperatur unterhalb 10° gehalten wird. Das Reaktionsgemisch wird nun während 18 Std. bei Raumtemperatur gerührt und schliesslich auf 500 ml gesättigte, wässrige Ammoniumchloridlösung gegossen. Die wässrige Phase wird mit Essigester extrahiert und die organische Phase mit gesättigter, wässriger Natriumchloridlösung gewaschen, über Natriumsulfat getrocknet und eingedampft. Das ölige Rohprodukt wird durch Flash-Chromatographie aufgetrennt (2,5 kg Kieselgel 60,40 - 63 µm, Laufmittel C).

Z-Cha$^{cx}$Val-OH ,

die weniger polare Komponente der Titelverbindung mit der gewünschten Konfiguration des an die Isopropylgruppe gebundenen C-Atoms (S-Konfiguration), wird als gelbes Oel erhalten. $R_f$ (I) = 0,20; $R_f$ (J) = 0,35.

h) HCl•H-Val-Tyr-OMe: 10,0 g Z-Val-Tyr-OMe werden in 200 ml Methanol und 24 ml 1 N HCl in Gegenwart von 1,0 g Palladium-Kohle (10 % Pd) bei Normaldruck und Raumtemperatur bis zur Sättigung hydriert. Das Reaktionsgemisch wird filtriert, und das Filtrat wird eingedampft und getrocknet. $R_f$ (K) = 0,64.

i)

Z-Cha$^{cx}$Val-Val-Tyr-OMe:

Eine Mischung aus 2,06 g HCl•H-Val-Tyr-OMe, 3,12 g Z-Cha$^{cx}$Val-OH , 1,74 g DCCI, 1,22 g HOBt, 0,75 g N-Methylmorpholin und 50 ml DMF wird 16 Std. bei Raumtemperatur gerührt. Der DCH wird abfiltriert,

das Filtrat eingeengt und am Hochvakuum getrocknet. Der Rückstand wird mittels Flash-Chromatographie (100 g Kieselgel 60, Laufmittel B) gereinigt. $R_f$ (A) = 0,36.

j)

H-Cha$^{C}$ Val-Val-Tyr-OMe:

3,6 g Z-Cha$^{cx}$Val-Val-Tyr-OMe werden in 80 ml Methanol-Wasser 9:1 in Gegenwart von 360 mg Palladium-Kohle (10 % Pd) bei Normaldruck und Raumtemperatur bis zur Sättigung hydriert. Das Reaktionsgemisch wird filtriert, und das Filtrat wird mit 30 ml Wasser verdünnt und für 5 Std. bei Raumtemperatur gerührt. Nach Abdampfen des Lösungsmittels wird die Titelverbindung aus Dioxan/Wasser 9:1 lyophilisiert. $R_f$ (H) = 0,10.

k)

Fmoc-Val-Cha$^{C}$ Val-Val-Tyr-OMe: 3,0 g (5,48 mMol) H-Cha$^{C}$ Val-Val-Tyr-OMe werden in 75 ml DMF gelöst. Zu dieser Lösung werden 3,69 g (7,12 mMol) Fmoc-Val-OTcp und dann 477 mg (6,03 mMol) Ethyldiisopropylamin zugegeben. Die Mischung wird 5 Std. bei Raumtemperatur gerührt, eingedampft und der Rückstand im Ultraschallbad in Diethylether digeriert. Das Produkt wird abfiltriert, mit Diethylether nachgewaschen und getrocknet. IR (KBr): 3300, 1695, 1645; FAB-MS: $(M+H)^+$ = 870; $R_f$ (L) = 0,5.

l)

H-Val-Cha$^{C}$ Val-Val-Tyr-OMe:

Zu einer Suspension von 3,62 g (4,17 mMol)

Fmoc-Val-Cha$^{C}$ Val-Val-Tyr-OMe

in 70 ml DMF werden 70 ml Piperidin zugegeben. Die Mischung wird 2 Std. bei Raumtemperatur gerührt, wobei eine klare Lösung entsteht. Die Lösung wird eingedampft, der Rückstand in Diisopropylether digeriert, abfiltriert, mit Diisopropylether nachgewaschen und getrocknet. IR (KBr): 2920, 1750, 1650; FAB-MS: $(M+H)^+$ = 647.

m) N-Benzyloxycarbonylthiomorpholin: Zu 1,03 g (10 mMol) Thiomorpholin in 30 ml Wasser werden innerhalb von 5 Min. 1,74 g (16 mMol) Chlorameisensäurebenzylester unter Rühren zugetropft. Die heterogene Mischung wird 30 Min. gerührt, wobei der pH durch kontinuierliche Zugabe von 1 N NaOH bei pH 9-0 konstant gehalten wird. Das Produkt wird mit Essigsäureethylester extrahiert, die organische Phase mit Wasser gewaschen, über $MgSO_4$ getrocknet, eingedampft und der Rückstand an Kieselgel chromatographiert. IR ($CH_2Cl_2$): 1682, 1415. $^1$H-NMR ($CDCl_3$): 2,50-2,76 (m,4H); 3,70-3,84 (m,4H); 5,15 (s,2H); 7,38 (s,5H).

n) N-Benzyloxycarbonylthiomorpholin-S,S-dioxid: Zu 510 mg (2,15 mMol) N-Benzyloxycarbonylthiomorpholin in 11 ml Methanol werden bei 5-8° innerhalb von 15 Min. 1,84 g (3,0 mMol) Oxone® (Kaliummonopersulfat-Tripelsalz, Fluka) in 10 ml Wasser zugetropft. Die Mischung wird 30 Min. bei Raumtemperatur gerührt, mit Wasser verdünnt und mit Essigsäureethylester extrahiert. Die organischen Extrakte werden über $MgSO_4$ getrocknet und eingedampft, wobei die Titelverbindung als kristalliner Festkörper anfällt. FAB-MS: $(M+H)^+$ = 270. $^1$H-NMR ($CDCl_3$): 2,9-3,1 (m,4H); 3,95-4,06 (m,4H); 5,15 (s,2H); 7,38 (s,5H).

o) Thiomorpholin-S,S-dioxid: 544 mg (2,02 mMol) N-BenzyloxycarbonylthiomorpholinS,S-dioxid werden in 30 ml Methanol in Gegenwart von Palladium auf Kohle (5 % Pd) 18 Stunden bei Raumtemperatur hydriert. Der Katalysator wird abfiltriert, das Filtrat eingedampft und der Rückstand aus Ether/Benzol unkristallisiert. Smp. 66-68°.

p) S,S-Dioxothiomorpholinocarbonylchlorid: 246 mg (1,82 mMol) Thiomorpholin-S,S-dioxid werden in 10 ml Toluol gelöst zu einer Lösung von 3,83 mMol Phosgen 20 % in Toluol bei 5-20° zugetropft. Die

Mischung wird 1 Std. bei Raumtemperatur gerührt. Das gebildete Hydrochlorid von Thiomorpholin-S,S-dioxid wird abfiltriert und mit Toluol nachgewaschen. Das Filtrat wird eingedampft, zuletzt am Hochvakuum, und die Titelverbindung direkt weiterverwendet. IR ($CH_2Cl_2$): 1725, 1320, 1120. $^1$H-NMR ($CDCl_3$): 3,09-3,20 (m,4H); 4,05-4,30 (m,4H).

Beispiel 2:

## Thiomorpholinocarbonyl-Val-Cha $^{C}$ Val-Val-Tyr-OMe

Zu 30 mg (0,046 mMol) H-Val-Cha $^{C}$ Val-Val-Tyr-OMe (Beispiel 11) gelöst in 1 ml DMF werden bei Raumtemperatur 23,2 µl Triethylamin und dann eine Lösung von 9,2 mg (0,055 mMol) Thiomorpholinocarbonylchlorid (hergestellt aus Thiomorpholin und Phosgen analog Beispiel 1p) in 1 ml Methylenchlorid zugegeben. Die Mischung wird 6 Std. bei Raumtemperatur gerührt, vollständig eingedampft und der Rückstand in wenig Diisopropylether digeriert. Das Rohprodukt wird durch Chromatographie an Kieselgel (Laufmittel F) gereinigt. $R_f$ (L) = 0,43; FAB-MS: $(M+H)^+$ = 776,7.

Beispiel 3:

## Morpholinocarbonyl-Val-Cha $^{C}$ Val-Val-Tyr-OMe

Zu 30 mg (0,046 mMol) H-Val-Cha $^{C}$ Val-Val-Tyr-OMe (Beispiel 11) gelöst in 1 ml DMF werden bei Raumtemperatur 23,2 µl Triethylamin und dann eine Lösung von 8,3 mg (0,055 mMol) Morpholinocarbonylchlorid (hergestellt aus Morpholin und Phosgen analog Beispiel 1p) in 1 ml Methylenchlorid zugegeben. Die Mischung wird 3 Std. bei Raumtemperatur gerührt, vollständig eingedampft und der Rückstand in wenig Diisopropylether digeriert. Das Rohprodukt wird durch Chromatographie an Kieselgel (Laufmittel F) gereinigt. $R_f$ (L) = 0,48; FAB-MS: $(M+H)^+$ = 760,6.

Beispiel 4:

## Z-Val-Cha $^{C}$ Val-Val-Tyr-OMe

Zu 40 mg H-Val-Cha $^{C}$ Val-Val-Tyr-OMe (Beispiel 1 l) in 1 ml DMF werden 25,8 ml Triethylamin und 10,6 µl Benzyloxycarbonylchlorid (Chlorameisensäurebenzylester, Z-Cl) zugegeben. Die Reaktionsmischung wird über Nacht bei Zimmertemperatur gerührt, dann eingedampft. Das Rohprodukt wird über Kieselgel mit Laufmittel F gereinigt. Die produkthaltigen Fraktionen werden eingedampft und der Rückstand mit Diisopropylether digeriert und getrocknet. IR (KBr): 3300, 2910, 1640; FAB-MS $(M+H)^+$ = 781.

Beispiel 5:

## Morpholinocarbonyl-Val-Cha$^{C}$Val -Val-Tyr-NH-n-propyl:

65 mg der Titelverbindung aus Beispiel 3 werden in 5 ml n-Propylamin gelöst und zuerst 18 Std. bei Raumtemperatur und dann 24 Std. im Bombenrohr bei 65 °C gerührt. Nach Einengen am Rotationsverdampfer unter vermindertem Druck wird das Rohprodukt durch Säulenchromatographie an Kieselgel (Laufmittel L) gereinigt. $R_f$(L) = 0,41; FAB-MS: $(M+H)^+$ = 787.

Beispiel 6:

## Morpholinocarbonyl-Val-Cha-$^{C}$Val-Val-Phe-$NH_2$:

Eine Lösung von 100 mg

H-Cha-$^{C}$Val-Val-Phe-$NH_2$

in 5 ml DMF wird nacheinander mit 2 ml einer Lösung von 53,5 mg Morpholinocarbonyl-Val-OH in DMF, 102,7 mg Bis-(2-oxo-3-oxazolidinyl)-phosphinsäurechlorid und 53,9 mg Triethylamin versetzt. Diese Lösung wird 30 Min. bei Raumtemperatur gerührt und dann bei 40 °C am Hochvakuum eingeengt. Der Rückstand wird in Diisopropylether digeriert. Das Rohprodukt wird durch Säulenchromatographie (Laufmittel L) an Kieselgel gereinigt. $R_f(L) = 0{,}43$; FAB-MS: $(M+H)^+ = 729$.

Die Ausgangsmaterialien werden folgendermassen hergestellt:

a) N-(5(S)-Azido-6-cyclohexyl-4(S)-tert-butyldimethylsilyloxy-2(S)-isopropyl-hexanoyl)-Val-Phe-$NH_2$: 192 mg 5(S)-Azido-6-cyclohexyl-4(S)-tert-butyldimethylsilyloxy-2(S)-isopropyl-hexansäure (J. Org. Chem. 54, 1178 (1989) werden in 4 ml DMF gelöst und nacheinander mit 227 mg Bis-(2-oxo-3-oxazolidinyl)-phosphinsäurechlorid, 69,3 mg HOBt und 154 µl N-Methylmorpholin versetzt. Nach 20 Min. Rühren bei Raumtemperatur wird zu diesem Gemisch 200 mg H-Val-Phe-$NH_2$ (Hydrochlorid, Bachem, Schweiz) gegeben. Nach 30 Min. Rühren bei Raumtemperatur wird an der Hochvakuumpumpe eingeengt, der Rückstand anschliessend in Methylenchlorid aufgenommen und zweimal mit gesättigter wässriger Natriumbicarbonatlösung gewaschen. Nach einmaligem Rückextrahieren der wässrigen Extrakte mit Methylenchlorid werden die vereinten organischen Phasen über Natriumsulfat getrocknet und eingedampft. Das Rohprodukt wird in n-Hexan/Essigester 1/1 (v/v) verrührt, abgenutscht und mit n-Hexan/Essigester nachgewaschen. $R_f(L) = 0{,}65$; FAB-MS: $(M+H)^+ = 657$.

b)

### H-Cha-$^{C}$Val-Val-Phe-$NH_2$:

860 mg der Titelverbindung von Beispiel 6 a) werden in 100 ml Methanol gelöst, mit 430 mg Palladiumkohle (5 % Pd) versetzt und mit Wasserstoff reduziert. Nach 4 Std. bei Raumtemperatur wird vom Katalysator abfiltriert, die Lösung eingeengt und der Rückstand in Ether digeriert. Nach Abfiltrieren und Trocknen erhält man die Titelverbindung. $R_f(L) = 0{,}83$; FAB-MS: $(M+H)^+ = 517$.

## Beispiel 7:

### Z-Asn-Cha-$^{C}$Val-Val-Tyr-OMe:

Eine Lösung von 274 mg

H-Cha-$^{C}$Val-Val-Tyr-OMe

(Beispiel 1 j)) in 5 ml DMF wird nacheinander mit 0,25 ml Ethyl-diisopropylamin und 232,4 mg Z-Asn-ONP (ONP = ortho-Nitrophenylester) in Portionen versetzt. Nach 1 Std. Rühren bei Raumtemperatur wird unter vermindertem Druck eingeengt, der Rückstand in Ether digeriert und abfiltriert. Dann wird der Feststoff nochmals in Methanol verrührt, abfiltriert und mit Methanol und Ether gewaschen. Nach Trocknen am Hochvakuum erhält man die Titelverbindung. $R_f(G) = 0{,}57$; FAB-MS: $(M+H^+) = 796$.

## Beispiel 8:

Morpholinocarbonyl-Asn-Cha-$^{c}$Val-Val-Tyr-OMe:

Analog Beispiel 3 werden 200

H-Asn-Cha-$^{c}$Val-Val-Tyr-OMe

mg in DMF mit 90,4 mg Morpholinocarbonylchlorid und 0,15 ml Triethylamin in die Titelverbindung überführt. Das Rohprodukt wird durch Chromatographie an Kieselgel (Laufmittel L) gereinigt. $R_f(G)$ = 0,45; FAB-MS: $(M+H)^+$ = 775.

Das Ausgangsmaterial wird wie folgt hergestellt:

a)

H-Asn-Cha-$^{c}$Val-Val-Tyr-OMe:

1022 mg der Titelverbindung aus Beispiel 7 werden in 150 ml Methanol gelöst, mit 200 mg Palladiumkohle (5 % Pd) versetzt und mit Wasserstoff reduziert. Nach 2,5 Std. bei Raumtemperatur wird vom Katalysator abfiltriert, die Lösung eingeengt und der Rückstand mit Ether im Ultraschallbad behandelt. Nach Abfiltrieren und nochmaligem Waschen mit Ether wird am Hochvakuum getrocknet. $R_f(L)$ = 0,07; FAB-MS: $(M+H)^+$ = 662.

Beispiel 9:

Morpholinocarbonyl-Asn-Cha-$^{c}$Val-Val-Tyr-NH-n-propyl:

80 mg der Titelverbindung aus Beispiel 8 werden in 5 ml N-Propylamin und 2 ml DMF bei 65 - 70 °C während 30 Std. im Bombenrohr gerührt. Nach Abkühlen und Einengen am Hochvakuum wird der Rückstand in Ether digeriert, abfiltriert und durch Säulenchromatographie (Laufmittel L) an Kieselgel gereinigt. $R_f(L)$ = 0,21; FAB-MS: $(M+H)^+$ = 802.

Beispiel 10:

Gelatine-Lösung

Eine wässrige Lösung von

S,S-Dioxothiomorpholinocarbonyl-Val-Cha$^{c}$Val-Val-Tyr-OMe

wird sterilfiltriert und unter Erwärmen mit einer sterilen Gelatinelösung, welche als Konservierungsmittel Phenol enthält, unter aseptischen Bedingungen so vermischt, dass 1,0 ml Lösung folgende Zusammensetzung hat:

| | |
|---|---|
| S,S-Dioxothiomorpholinocarbonyl-Val-Cha$^{c}$Val-Val-Tyr-OMe | 3 mg |
| Gelatine | 150,0 mg |
| Phenol | 4,7 mg |
| dest. Wasser bis zu | 1,0 ml |

Die Mischung wird unter aseptischen Bedingungen in Vialen zu 1,0 ml abgefüllt.

Beispiel 11:

Sterile Trockensubstanz zur Injektion

Man löst 5 mg

S,S-Dioxothiomorpholinocarbonyl-Val-Cha $^{C}$Val-Val-Tyr-OMe

in 1 ml einer wässrigen Lösung mit 20 mg Mannit. Die Lösung wird sterilfiltriert und unter aseptischen Bedingungen in eine 2 ml Ampulle gefüllt, tiefgekühlt und lyophilisiert. Vor dem Gebrauch wird das Lyophilisat in 1 ml destilliertem Wasser oder 1 ml physiologischer Salzlösung gelöst. Die Lösung wird intramuskulär oder intravenös angewendet. Diese Formulierung kann auch in Doppelkammerspritzampullen abgefüllt werden.

Beispiel 12:

Nasenspray

In einer Mischung von 3,5 ml Myglyol 812® und 0,08 g Benzylalkohol werden 500 mg fein gemahlenes (<5,0 µm)

S,S-Dioxothiomorpholinocarbonyl-Val-Cha $^{C}$Val-Val-Tyr-OMe

suspendiert. Diese Suspension wird in einen Behälter mit Dosierventil eingefüllt. Es werden 5,0 g Freon® 12 unter Druck durch das Ventil in den Behälter abgefüllt. Durch Schütteln wird das Freon® in der Myglyol-Benzylalkoholmischung gelöst. Dieser Spraybehälter enthält ca. 100 Einzeldosen, die einzeln appliziert werden können.

Beispiel 13:

Lacktabletten

Für die Herstellung von 10 000 Tabletten enthaltend je 100 mg Wirkstoff werden folgende Bestandteile verarbeitet:

| | |
|---|---|
| S,S-Dioxothiomorpholinocarbonyl-Val-Cha $^{C}$Val-Val-Tyr-OMe | 1000 g |
| Maisstärke | 680 g |
| Kolloidale Kieselsäure | 200 g |
| Magnesiumstearat | 20 g |
| Stearinsäure | 50 g |
| Natriumcarboxymethylstärke | 250 g |
| Wasser | q.s. |

Ein Gemisch des

S,S-Dioxothiomorpholinocarbonyl-Val-Cha $^{C}$Val-Val-Tyr-OMe,

50g Maisstärke und der kolloidalen Kieselsäure wird mit Stärkekleister aus 250 g Maisstärke und 2,2 kg entmineralisiertem Wasser zu einer feuchten Masse verarbeiter. Diese wird durch ein Sieb von 3 mm Maschenweite getrieben und bei 45° während 30 Min. im Wirbelschichttrockner getrocknet. Das trockene Granulat wird durch ein Sieb von 1 mm Maschenweite gedrückt, mit einer vorher gesiebten Mischung (1 mm Sieb) von 330 g Maisstärke, des Magnesiumstearats, der Stearinsäure und der Natriumcarboxymethylstärke gemischt und zu schwach gewölbten Tabletten verpresst.

Die Presslinge werden in einem Dragierkessel von 45 cm Durchmesser mit einer Lösung von 20 g Schellack und 40 g Hydroxypropylmethylcellulose (niedere Viskosität) in 110 g Methanol und 1350 g Methylenchlorid durch gleichmässiges Aufsprühen während 30 Min. überzogen; dabei wird durch gleichzeitiges Einblasen von Luft von 60° getrocknet.

Anstelle der in den Beispielen 5-8 genannten Wirkstoffe kann man in diesen Beispielen auch dieselbe Menge eines anderen Wirkstoffes der vorangehenden Beispiele verwenden.

Beispiel 14:

Hemmung der isolierten HIV-1 gag-Protease

10 µl einer Lösung von HIV-1 gag-Protease (Aceton-Extrakt einer in E. coli exprimierten gag-Protease gemäss J. Hansen et al., The EMBO Journal 7, 1785 (1988)) und 190 µl β-Morpholinoethansulfonsäure-Pufferlösung pH 6 enthaltend 0,01 % 2-Amino-4-nitrophenol als internem Standard werden bei 37° vorinkubiert. Dann werden gleichzeitig 10 µl einer 0,24 mM DMSO-Lösung des Substrats H-Arg-Arg-Ser-Asn-Gln-Val-Ser-Gln-Asn-Tyr-Pro-Ile-Val-Gln-Asn-Ile-Gln-Gly-Arg-Arg-OH (Icosapeptid gemäss J. Schneider et al., Cell 54, 363 (1988), hergestellt auf einem automatisierten Peptidsynthesegerät mit Fmoc-geschützten Aminosäurebausteinen) und 10 µl einer DMSO-Lösung der auf Hemmwirkung zu untersuchenden Verbindung in einer Konzentration von 22 x $10^{-4}$ M oder 22 x $10^{-6}$ M zugegeben. Nach einer Stunde werden 50 µl Reaktionslösung entnommen, mit 5 µl 0,3 M Perchlorsäure versetzt und zentrifugiert. Die Menge an unverbrauchtem Substrat und die Spaltprodukte in der überstehenden Lösung werden mit HPLC bestimmt und daraus die prozentuale Hemmung bei $10^{-4}$ M und $10^{-6}$ M berechnet.

Für die HPLC-Analyse wird eine 125 x 4,6 mm reversed phase $C_{18}$ Nucleosil® 5 µ Säule verwendet; Gradient 10 % Acetonitril/0,1 % Trifluoressigsäure in Wasser → 25 % Acetonitril/0,08 % Trifluoressigsäure in Wasser in 30 Min., Durchflussrate 1,5 ml/Min.

Die Verbindungen der vorstehenden Beispiele weisen bei Konzentrationen von $10^{-4}$ M eine Hemmwirkung von über 80 % und bei $10^{-6}$ M eine Hemmwirkung von über 50 % auf.

Beispiel 15:

Schutz gegen HIV-Infektion im Zelltest

Die verwendete Zellinie MT-2 ist eine menschliche T-Zell-Leukämie, die mit HTLV-1 transformiert ist und HTLV-1 kontinuierlich produziert, was die Zellen extrem empfindlich für den zytopathogenen Effekt von HIV macht (Science 229, 563 (1985)). Die MT-2 Zellen werden in RPMI 1640 Medium enthaltend 12 % hitze-inaktiviertes fötales Kälberserum (Seromed Biochrom KG, Berlin, BRD), Glutamin und Standard-Antibiotika kultiviert. Die Zellen werden bei 37° in befeuchteter Luft mit 5 % $CO_2$ gehalten und in der logarithmischen Wachstumsphase für den Zelltest verwendet.

HIV LAV-03 (AIDS Research and Reference Reagent Program, NIH, Bethesda, MD, USA) wird in A 3.01-Zellen gezüchtet. Der Titer wird im Reverse Transcriptase Assay bestimmt. Für die verwendete Charge beträgt er 2 x $10^7$ IU/ml.

40'000 exponentiell wachsende MT-2 Zellen in 50 µl Kulturmedium werden in jede der Vertiefungen einer 96 Rundboden-Titerplatte gegeben. Die zu untersuchenden Verbindungen werden in vorgegebener Konzentration in 50 µl Kulturmedium beigefügt und unmittelbar danach HIV in 100 µl Kulturmedium zugegeben. Zu Vergleichsproben wird 100 µl Kulturmedium ohne HIV zugegeben. Die Titerplatten werden sechs Tage inkubiert. Danach wird die Lebensfähigkeit der HIV-infizierten und der Vergleichs-Zellen im MTT-Assay geprüft: Der MTT-Assay beruht auf der Reduktion von gelbgefärbtem 3-(4,5-Dimethylthiazol-2-yl)-2,5-diphenyltetrazolium-bromid (MTT, Sigma, St.Louis, USA) durch mitochondrische Dehydrogenasen von metabolisch aktiven Zellen zu einem blauen Formazan, das spektrophotometrisch bei 540 nm gemessen werden kann (J. Virological Methods 20, 309 (1988)). Die Lebensfähigkeit von Vergleichszellen und HIV-infizierten Zellen wird ebenfalls mikroskopisch in einem Hämozytometer nach der Trypanblau-Ausschlussmethode bestimmt.

Die untersuchten Verbindungen der vorstehenden Beispiele weisen bei Konzentrationen von $10^{-5}$ mol/l Schutzwirkung gegen HIV-Infektion auf.

**Patentansprüche**

1. Verbindungen der Formel

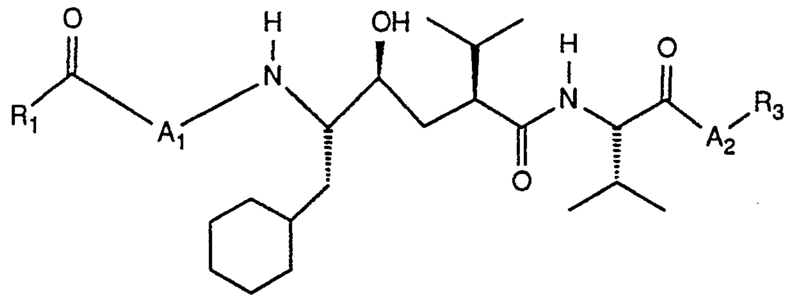

(I),

worin $R_1$ Diniederalkylamino, Pyrrolidino, Piperidino, Morpholino, Thiomorpholino, S,S-Dioxothiomorpholino, Piperazino, N-Niederalkylpiperazino, Benzyloxy oder im Phenylring durch Niederalkyl oder Niederalkoxy substituiertes Benzyloxy, $A_1$ ein bivalentes Radikal aus einer der Aminosäuren Valin oder Asparagin, das N-terminal an die Gruppe -(C=O) und C-terminal an die Gruppe -NH gebunden ist, $A_2$ ein bivalentes Radikal aus einer der Aminosäuren Tyrosin oder Phenylalanin, das N-terminal an die Gruppe -(C=O und C-terminal an die Gruppe -NH gebunden ist, und $R_3$ $OR_2$ oder $NHR_2$, worin $R_2$ Wasserstoff oder Niederalkyl ist, bedeuten, ferner Salze dieser Verbindungen.

2. Verbindungen gemäss Anspruch 1 der Formel I, worin $R_1$ Diniederalkylamino, Piperidino, Morpholino, Thiomorpholino, S,S-Dioxothiomorpholino oder Benzyloxy, $A_1$ den bivalenten Rest der Aminosäure Valin, $A_2$ den bivalenten Rest der Aminosäure Tyrosin, und $R_3$ $OR_2$, worin $R_2$ Niederalkyl ist, bedeuten.

3. Verbindungen gemäss Anspruch 1 der Formel I, worin $R_1$ Diniederalkylamino, Piperidino, Morpholino, Thiomorpholino, S,S-Dioxothiomorpholino oder Benzyloxy, $A_1$ den bivalenten Rest der Aminosäure Asparagin oder Valin, $A_2$ den bivalenten Rest der Aminosäure Phenylalanin oder Tyrosin, und $R_3$ $OR_2$, worin $R_2$ Niederalkyl ist, oder $NHR_2$, worin $R_2$ Wasserstoff oder Niederalkyl ist, bedeuten.

4. Verbindungen gemäss Anspruch 1 der Formel I, worin $R_1$ Morpholino, Thiomorpholino, S,S-Dioxothiomorpholino oder Benzyloxy, $A_1$ den bivalenten Rest der Aminosäure Valin, $A_2$ den bivalenten Rest der Aminosäure Tyrosin, und $R_3$ $OR_2$, worin $R_2$ Niederalkyl ist, bedeuten.

5. Verbindungen gemäss Anspruch 1 der Formel I, worin $R_1$ Morpholino oder Benzyloxy, $A_1$ den bivalenten Rest der Aminosäure Asparagin, $A_2$ den bivalenten Rest der Aminosäure Tyrosin, und $R_3$ $OR_2$, worin $R_2$ Niederalkyl ist, bedeuten.

6. Verbindungen gemäss Anspruch 1 der Formel I, worin $R_1$ Morpholino, $A_1$ den bivalenten Rest der Aminosäure Valin oder Asparagin, $A_2$ den bivalenten Rest der Aminosäure Phenylalanin oder Tyrosin, und $R_3$ $NHR_2$, worin $R_2$ Wasserstoff oder Niederalkyl ist, bedeuten.

7. Die Verbindung gemäss Anspruch 1 der Formel I, worin $R_1$ S,S-Dioxothiomorpholino, $A_1$ den bivalenten Rest der Aminosäure Valin, $A_2$ den bivalenten Rest der Aminosäure Tyrosin und $R_3$ Methoxy bedeuten.

8. Die Verbindung gemäss Anspruch 1 der Formel I, worin $R_1$ Thiomorpholino, $A_1$ den bivalenten Rest der Aminosäure Valin, $A_2$ den bivalenten Rest der Aminosäure Tyrosin und $R_3$ Methoxy bedeuten.

9. Die Verbindung gemäss Anspruch 1 der Formel I, worin $R_1$ Morpholino, $A_1$ den bivalenten Rest der Aminosäure Valin, $A_2$ den bivalenten Rest der Aminosäure Tyrosin und $R_3$ Methoxy bedeuten.

10. Die Verbindung gemäss Anspruch 1 der Formel I, worin $R_1$ Benzyloxy, $A_1$ den bivalenten Rest der

Aminosäure Valin, $A_2$ den bivalenten Rest der Aminosäure Tyrosin und $R_3$ Methoxy bedeuten.

11. Die Verbindung gemäss Anspruch 1 der Formel I, worin $R_1$ Morpholino, $A_1$ den bivalenten Rest der Aminosäure Valin, $A_2$ den bivalenten Rest der Aminosäure Tyrosin und $R_3$ n-Propylamino bedeuten.

12. Die Verbindung gemäss Anspruch 1 der Formel I, worin $R_1$ Morpholino, $A_1$ den bivalenten Rest der Aminosäure Valin, $A_2$ den bivalenten Rest der Aminosäure Phenylalanin und $R_3$ Amino bedeuten.

13. Die Verbindung gemäss Anspruch 1 der Formel I, worin $R_1$ Benzyloxy, $A_1$ den bivalenten Rest der Aminosäure Asparagin, $A_2$ den bivalenten Rest der Aminosäure Tyrosin und $R_3$ Methoxy bedeuten.

14. Die Verbindung gemäss Anspruch 1 der Formel I, worin $R_1$ Morpholino, $A_1$ den bivalenten Rest der Aminosäure Asparagin, $A_2$ den bivalenten Rest der Aminosäure Tyrosin und $R_3$ Methoxy bedeuten.

15. Die Verbindung gemäss Anspruch 1 der Formel I, worin $R_1$ Morpholino, $A_1$ den bivalenten Rest der Aminosäure Asparagin, $A_2$ den bivalenten Rest der Aminosäure Tyrosin und $R_3$ n-Propylamino bedeuten.

16. Pharmazeutische Präparate enthaltend Verbindungen gemäss einem der Ansprüche 1 bis 15 oder ein pharmazeutisch annehmbares Salz davon zusammen mit einem pharmazeutisch geeigneten Trägermaterial.

17. Die in den Ansprüchen 1 bis 15 genannten Verbindungen zur Anwendung in einem Verfahren zur therapeutischen Behandlung des menschlichen oder tierischen Körpers.

18. Verwendung der in den Ansprüchen 1 bis 15 genannten Verbindungen als retrovirale Proteasehemmer.

19. Verfahren zur Herstellung von den in Anspruch 1 genannten Verbindungen der Formel I, worin $R_1$, $A_1$, $A_2$ und $R_3$ die in Anspruch 1 angegebenen Bedeutungen haben, und deren Salzen, dadurch gekennzeichnet, dass man ein Fragment einer Verbindung der Formel I mit einer endständigen Carboxygruppe oder ein reaktionsfähiges Säurederivat dieses Fragments mit einem zur Verbindung der Formel I komplementären Fragment mit einer freien Aminogruppe oder einem reaktionsfähigen Derivat davon mit aktivierter Aminogruppe, wobei in den Reaktionskomponenten vorhandene funktionelle Gruppen mit Ausnahme der an der Reaktion teilnehmenden Gruppen gegebenenfalls in geschützter Form vorliegen, unter Bildung einer Amidbindung kondensiert, und gewünschtenfalls in einer erhältlichen Verbindung vorhandene Schutzgruppen abspaltet und/oder eine erhältliche Verbindung der Formel I mit einer salzbildenden Gruppe in ihr Salz oder ein erhältliches Salz in die freie Verbindung oder in ein anderes Salz überführt und/oder gegebenenfalls erhältliche Isomerengemische auftrennt und/oder eine erfindungsgemässe Verbindung der Formel I in eine andere erfindungsgemässe Verbindung der Formel I umwandelt.

**Patentansprüche für folgende Vertragsstaaten: ES, GR**

1. Verfahren zur Herstellung von Verbindungen der Formel

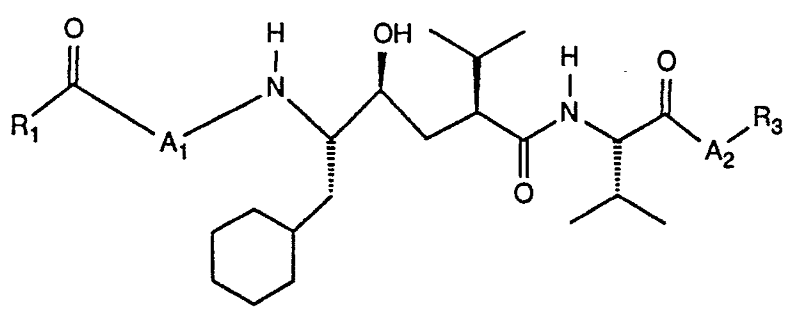

(I),

worin $R_1$ Diniederalkylamino, Pyrrolidino, Piperidino, Morpholino, Thiomorpholino, S,S-Dioxothiomorpholino, Piperazino, N-Niederalkylpiperazino, Benzyloxy oder im Phenylring durch Niederalkyl oder Niederalkoxy substituiertes Benzyloxy, $A_1$ ein bivalentes Radikal aus einer der Aminosäuren Valin oder Asparagin, das N-terminal an die Gruppe -(C=O) und C-terminal an die Gruppe -NH gebunden ist, $A_2$ ein bivalentes Radikal aus einer der Aminosäuren Tyrosin oder Phenylalanin, das N-terminal an die Gruppe -(C=O) und C-terminal an die Gruppe -NH gebunden ist, und $R_3$ $OR_2$ oder $NHR_2$, worin $R_2$ Wasserstoff oder Niederalkyl ist, bedeuten, ferner von Salzen dieser Verbindungen, dadurch gekennzeichnet, dass man

ein Fragment einer Verbindung der Formel I mit einer endständigen Carboxygruppe oder ein reaktionsfähiges Säurederivat dieses Fragments mit einem zur Verbindung der Formel I komplementären Fragment mit einer freien Aminogruppe oder einem reaktionsfähigen Derivat davon mit aktivierter Aminogruppe, wobei in den Reaktionskomponenten vorhandene funktionelle Gruppen mit Ausnahme der an der Reaktion teilnehmenden Gruppen gegebenenfalls in geschützter Form vorliegen, unter Bildung einer Amidbindung kondensiert,

und gewünschtenfalls in einer erhältlichen Verbindung vorhandene Schutzgruppen abspaltet und/oder eine erhältliche Verbindung der Formel I mit einer salzbildenden Gruppe in ihr Salz oder ein erhältliches Salz in die freie Verbindung oder in ein anderes Salz überführt und/oder gegebenenfalls erhältliche Isomerengemische auftrennt und/oder eine erfindungsgemässe Verbindung der Formel I in eine andere erfindungsgemässe Verbindung der Formel I umwandelt.

2. Verfahren zur Herstellung von Verbindungen der Formel I gemäss Anspruch 1, dadurch gekennzeichnet, dass man die Ausgangsverbindungen so wählt, dass man eine Verbindung der Formel I herstellt, worin $R_1$ Diniederalkylamino, Piperidino, Morpholino, Thiomorpholino, S,S-Dioxothiomorpholino oder Benzyloxy, $A_1$ den bivalenten Rest der Aminosäure Valin, $A_2$ den bivalenten Rest der Aminosäure Tyrosin, und $R_3$ $OR_2$, worin $R_2$ Niederalkyl ist, bedeuten.

3. Verfahren zur Herstellung von Verbindungen der Formel I gemäss Anspruch 1, dadurch gekennzeichnet, dass man die Ausgangsverbindungen so wählt, dass man eine Verbindung der Formel I herstellt, worin $R_1$ Diniederalkylamino, Piperidino, Morpholino, Thiomorpholino, S,S-Dioxothiomorpholino oder Benzyloxy, $A_1$ den bivalenten Rest der Aminosäure Asparagin oder Valin, $A_2$ den bivalenten Rest der Aminosäure Phenylalanin oder Tyrosin, und $R_3$ $OR_2$, worin $R_2$ Niederalkyl ist, oder $NHR_2$, worin $R_2$ Wasserstoff oder Niederalkyl ist, bedeuten.

4. Verfahren zur Herstellung von Verbindungen der Formel I gemäss Anspruch 1, dadurch gekennzeichnet, dass man die Ausgangsverbindungen so wählt, dass man eine Verbindung der Formel I herstellt, worin $R_1$ Morpholino, Thiomorpholino, S,S-Dioxothiomorpholino oder Benzyloxy, $A_1$ den bivalenten Rest der Aminosäure Valin, $A_2$ den bivalenten Rest der Aminosäure Tyrosin, und $R_3$ $OR_2$, worin $R_2$ Niederalkyl ist, bedeuten.

5. Verfahren zur Herstellung von Verbindungen der Formel I gemäss Anspruch 1, dadurch gekennzeichnet, dass man die Ausgangsverbindungen so wählt, dass man eine Verbindung der Formel I herstellt, worin $R_1$ Morpholino oder Benzyloxy, $A_1$ den bivalenten Rest der Aminosäure Asparagin, $A_2$ den bivalenten Rest der Aminosäure Tyrosin, und $R_3$ $OR_2$, worin $R_2$ Niederalkyl ist, bedeuten.

6. Verfahren zur Herstellung von Verbindungen der Formel I gemäss Anspruch 1, dadurch gekennzeichnet, dass man die Ausgangsverbindungen so wählt, dass man eine Verbindung der Formel I herstellt, worin $R_1$ Morpholino, $A_1$ den bivalenten Rest der Aminosäure Valin oder Asparagin, $A_2$ den bivalenten Rest der Aminosäure Phenylalanin oder Tyrosin, und $R_3$ $NHR_2$, worin $R_2$ Wasserstoff oder Niederalkyl ist, bedeuten.

7. Verfahren zur Herstellung von Verbindungen der Formel I gemäss Anspruch 1, dadurch gekennzeichnet, dass man die Ausgangsverbindungen so wählt, dass man eine Verbindung der Formel I herstellt, worin $R_1$ S,S-Dioxothiomorpholino, $A_1$ den bivalenten Rest der Aminosäure Valin, $A_2$ den bivalenten Rest der Aminosäure Tyrosin und $R_3$ Methoxy bedeuten.

8. Verfahren zur Herstellung von Verbindungen der Formel I gemäss Anspruch 1, dadurch gekennzeichnet, dass man die Ausgangsverbindungen so wählt, dass man eine Verbindung der Formel I herstellt,

worin $R_1$ Thiomorpholino, $A_1$ den bivalenten Rest der Aminosäure Valin, $A_2$ den bivalenten Rest der Aminosäure Tyrosin und $R_3$ Methoxy bedeuten.

9. Verfahren zur Herstellung von Verbindungen der Formel I gemäss Anspruch 1, dadurch gekennzeichnet, dass man die Ausgangsverbindungen so wählt, dass man eine Verbindung der Formel I herstellt, worin $R_1$ Morpholino, $A_1$ den bivalenten Rest der Aminosäure Valin, $A_2$ den bivalenten Rest der Aminosäure Tyrosin und $R_3$ Methoxy bedeuten.

10. Verfahren zur Herstellung von Verbindungen der Formel I gemäss Anspruch 1, dadurch gekennzeichnet, dass man die Ausgangsverbindungen so wählt, dass man eine Verbindung der Formel I herstellt, worin $R_1$ Benzyloxy, $A_1$ den bivalenten Rest der Aminosäure Valin, $A_2$ den bivalenten Rest der Aminosäure Tyrosin und $R_3$ Methoxy bedeuten.

11. Verfahren zur Herstellung von Verbindungen der Formel I gemäss Anspruch 1, dadurch gekennzeichnet, dass man die Ausgangsverbindungen so wählt, dass man eine Verbindung der Formel I herstellt, worin $R_1$ Morpholino, $A_1$ den bivalenten Rest der Aminosäure Valin, $A_2$ den bivalenten Rest der Aminosäure Tyrosin und $R_3$ n-Propylamino bedeuten.

12. Verfahren zur Herstellung von Verbindungen der Formel I gemäss Anspruch 1, dadurch gekennzeichnet, dass man die Ausgangsverbindungen so wählt, dass man eine Verbindung der Formel I herstellt, worin $R_1$ Morpholino, $A_1$ den bivalenten Rest der Aminosäure Valin, $A_2$ den bivalenten Rest der Aminosäure Phenylalanin und $R_3$ Amino bedeuten.

13. Verfahren zur Herstellung von Verbindungen der Formel I gemäss Anspruch 1, dadurch gekennzeichnet, dass man die Ausgangsverbindungen so wählt, dass man eine Verbindung der Formel I herstellt, worin $R_1$ Benzyloxy, $A_1$ den bivalenten Rest der Aminosäure Asparagin, $A_2$ den bivalenten Rest der Aminosäure Tyrosin und $R_3$ Methoxy bedeuten.

14. Verfahren zur Herstellung von Verbindungen der Formel I gemäss Anspruch 1, dadurch gekennzeichnet, dass man die Ausgangsverbindungen so wählt, dass man eine Verbindung der Formel I herstellt, worin $R_1$ Morpholino, $A_1$ den bivalenten Rest der Aminosäure Asparagin, $A_2$ den bivalenten Rest der Aminosäure Tyrosin und $R_3$ Methoxy bedeuten.

15. Verfahren zur Herstellung von Verbindungen der Formel I gemäss Anspruch 1, dadurch gekennzeichnet, dass man die Ausgangsverbindungen so wählt, dass man eine Verbindung der Formel I herstellt, worin $R_1$ Morpholino, $A_1$ den bivalenten Rest der Aminosäure Asparagin, $A_2$ den bivalenten Rest der Aminosäure Tyrosin und $R_3$ n-Propylamino bedeuten.

16. Verfahren zur Herstellung von pharmazeutischen Präparaten enthaltend Verbindungen der Formel I gemäss Anspruch 1 oder ein pharmazeutisch annehmbares Salz davon zusammen mit einem pharmazeutisch geeigneten Trägermaterial.